# EUROPEAN PATENT APPLICATION

(11) **EP 4 603 106 A1**
(43) Date of publication of application: **20.08.2025**
(21) Application number: 24382172.5
(22) Date of filing: 19.02.2024
(51) Int. Cl.: A61K 38/45, A61K 35/15, A61K 48/00, A61P 9/10, C12N 5/0784

(54) **POPULATIONS OF SMALL EXTRACELLULAR VESICLES FOR USE IN THE TREATMENT OR PREVENTION OF ISCHEMIA**

(71) Applicant: Universidade de Santiago de Compostela, 15782 Santiago de Compostela (ES); Fundación Instituto de Investigación Sanitaria de Santiago de Compostela, 15706 Santiago de Compostela (ES); Servizo Galego de Saude, 15703 Santiago de Compostela (ES)
(72) Inventor: LÓPEZ PÉREZ, Miguel Antonio, 15782 Santiago de Compostela (ES); SOBRINO MOREIRAS, Tomás, 15706 Santiago de Compostela (ES); OURO VILLASANTE, Alberto, 15706 Santiago de Compostela (ES)
(74) Representative: Hoffmann Eitle

(57) **Abstract**

The present invention provides a population of small extracellular vesicles (sEVs) comprising at least one polynucleotide encoding a dominant negative AMP-activated protein kinase alpha 2 (AMPKα2-DN) mutant protein. Uses in medicine, particularly in the treatment or prevention of ischemia are also provided.

## Description

### TECHNICAL FIELD OF THE INVENTION

The present invention relates to the field of nanobiomedicine. Particularly, the present invention relates to the use of small extracellular vesicles for the treatment or prevention of ischemic processes.

### BACKGROUND OF THE INVENTION

Stroke is one of the leading causes of mortality and morbidity worldwide. In addition, most of the survivors suffer some degree of long-term adult disability. According to the WHO, the global average incidence of cerebrovascular diseases is about 200 new cases per 100,000 habitants. Due to the aging of the population, the incidence of stroke is progressively growing, since its prevalence is higher in people over 65 years old. Several risk factors have been associated with ischemic stroke such as age, high blood pressure, heart disease, diabetes, hyperlipidemia, smoking or obesity.

During the stroke, part of the tissue is subjected to an ischemic process. Ischemia is an interruption of blood flow to a tissue or organ, which leads to tissue hypoxia and anoxia. During ischemia, anaerobic glycolysis, adenosine triphosphate (ATP) hydrolysis, and release of protons from acidic organelles cause pH in solid tissues to decrease by one unit or more (≥10-fold increase of hydrogen ion concentration).

Current treatments for ischemic stroke include improving blood flow to the affected tissue/organ. Treatment may include medications (such as nitroglycerine, beta-blockers, and calcium channel blockers), a procedure to open blocked arteries (angioplasty) or bypass surgery. However, right before, during, and right after an ischemic episode, it is crucial to treat the patient quickly to avoid sequels and complications. The most effective treatment requires expertise that can only be applied in the hospital, while it may be too late for the patient. Thus, effective and off-the-shelf treatments need to be developed to treat ischemic patients as soon as the process starts, in order to avoid permanent loss of functionality.

Extracellular vesicles (EVs) are heterogeneous populations of naturally occurring nano to micro-sized membrane vesicles released by essentially all cell types. It has been shown that EVs possess therapeutic potential through the reprogramming of target cells, affording modulation of cellular processes and secretomes - the molecules secreted by cells - and eventually favoring tissue repair after reprogramming of target cells.

The present invention provides the use of extracellular vesicles as therapeutic vectors that are administered systemically to deliver a plasmid encoding for a dominant negative isoform of AMPKα2 for use in the treatment or prevention of ischemia.

### DESCRIPTION OF THE FIGURES

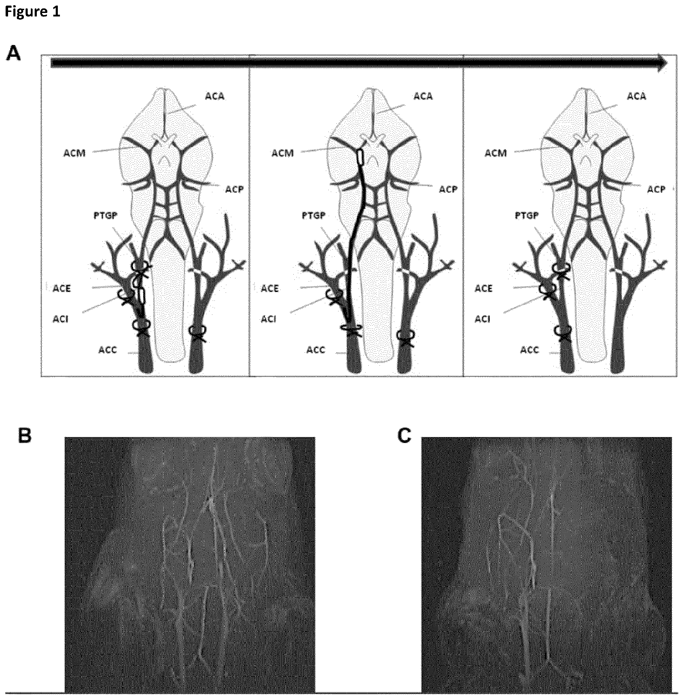

**Fig. 1** **tMCAO model**. A) Schematic representation of occlusion of the middle cerebral artery. B) MRI angiography showing the physiological vascularity of a healthy mouse. C) Angiography of a mouse subjected to tMCAO.

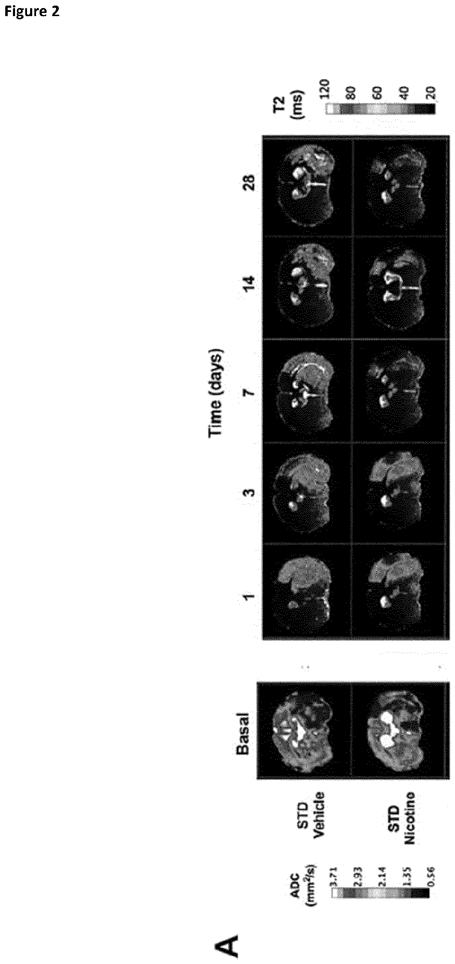

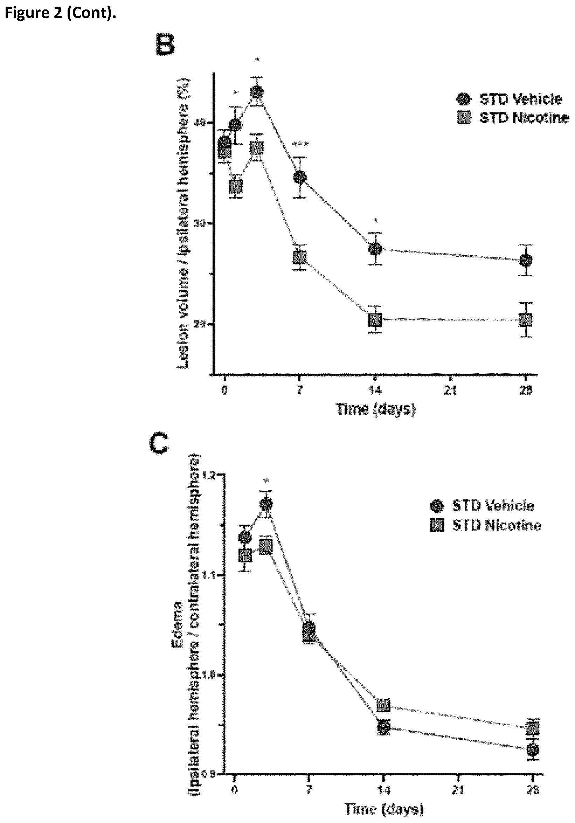

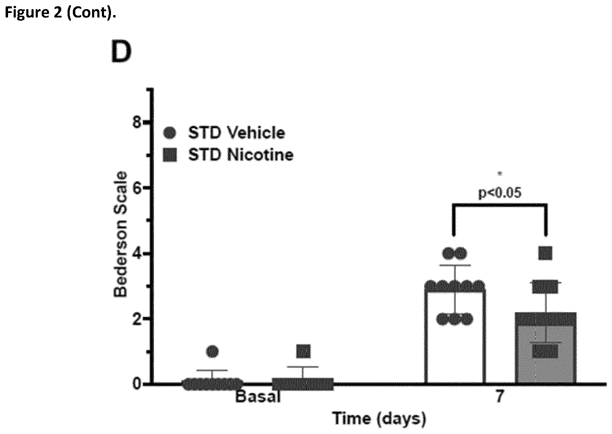

**Fig. 2** **Effect of nicotine on STD ischemic rats**. A) Ischemic lesion and edema volumes were assessed using MRI. Representative ADC maps (which were obtained from DWI) showing initial homogeneous lesion volumes of STD rats non-treated (vehicle) or treated with nicotine. T2 relaxation maps (which were obtained from T2-weighted images) on each row correspond to one of 2D slices of the same animals within each group at days 1, 3, 7, 14 and 28 after ischemia onset. B and C) Time course of lesion volumes in STD rats treated or non-treated (vehicle) with nicotine. D) Time course of edema volumes in STD rats treated or non-treated (vehicle) with nicotine. Data are shown as mean±SEM. *p<0.05; **p<0.01 compared to the vehicle STD group; #p<0.05 compared to the vehicle HFD group; using the two-way analysis of variance (ANOVA) statistical test; (n=18 for the vehicle STD group, n=19 for the nicotine STD group, in the follow-ups at 24 hours, 72 hours and 7 days; and n=10, n=10, n=8, respectively, in the follow-ups at 14 and 28 days).

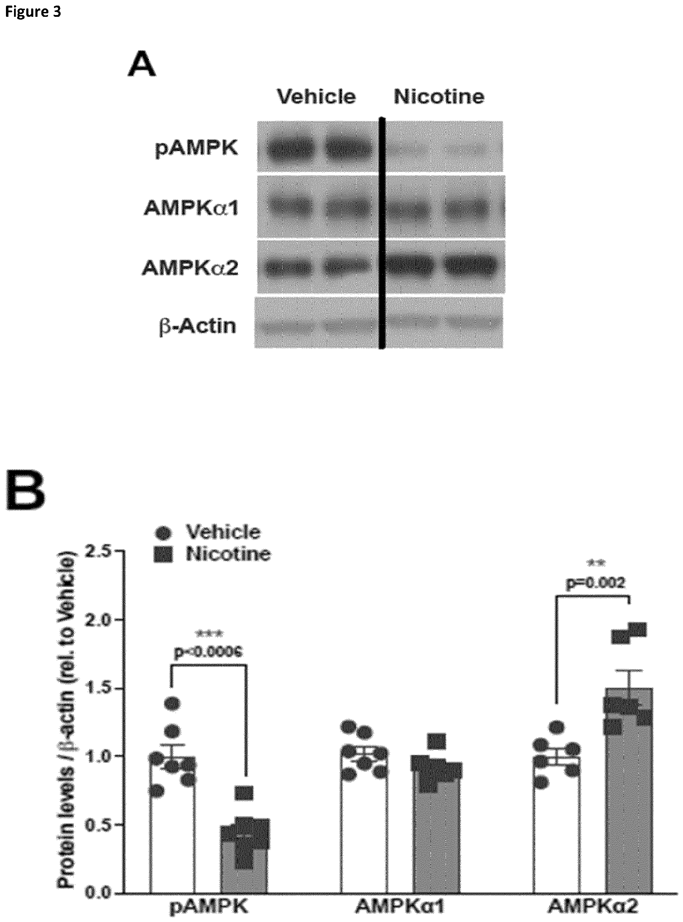

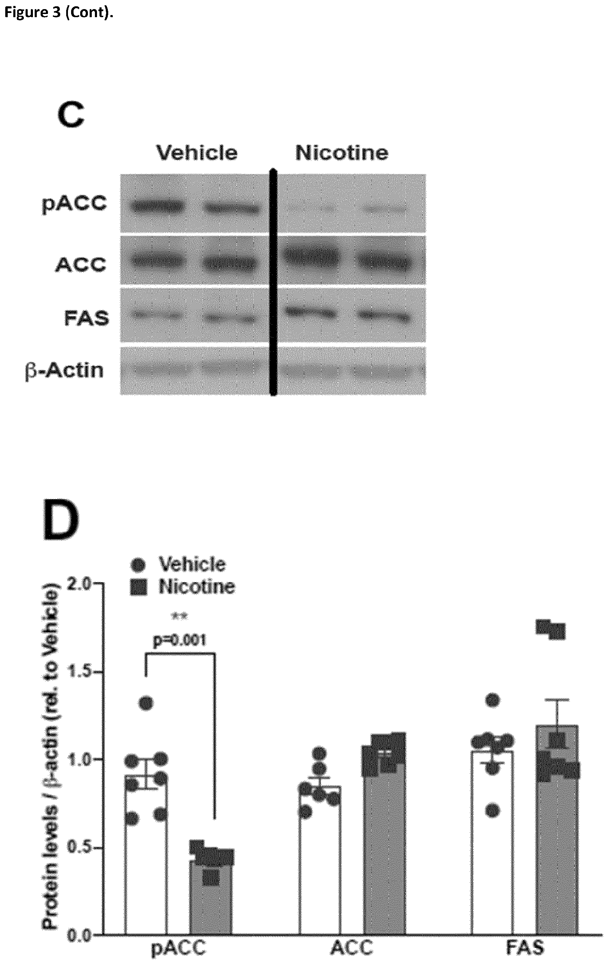

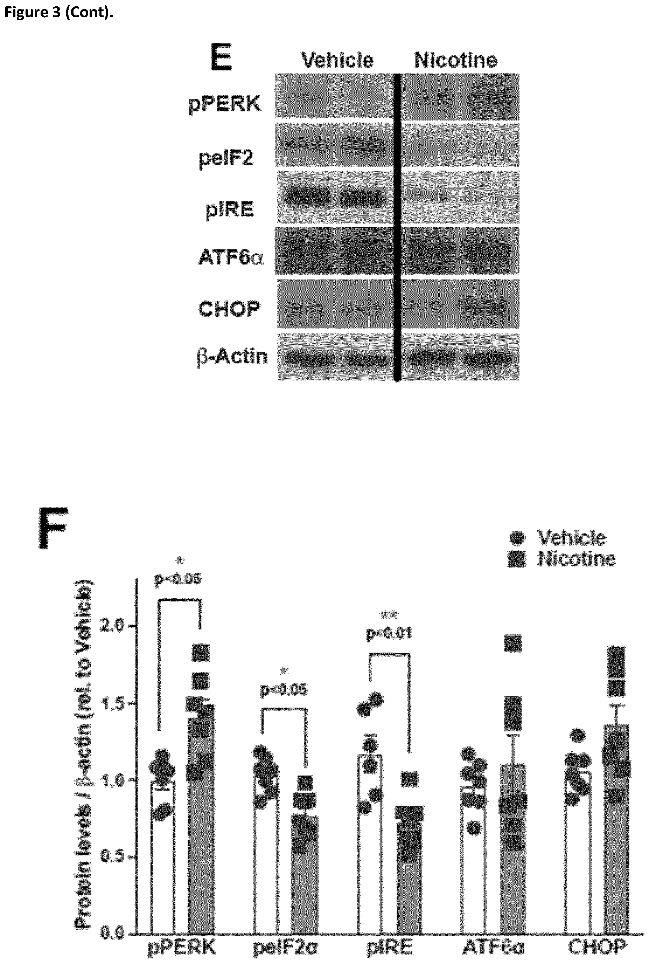

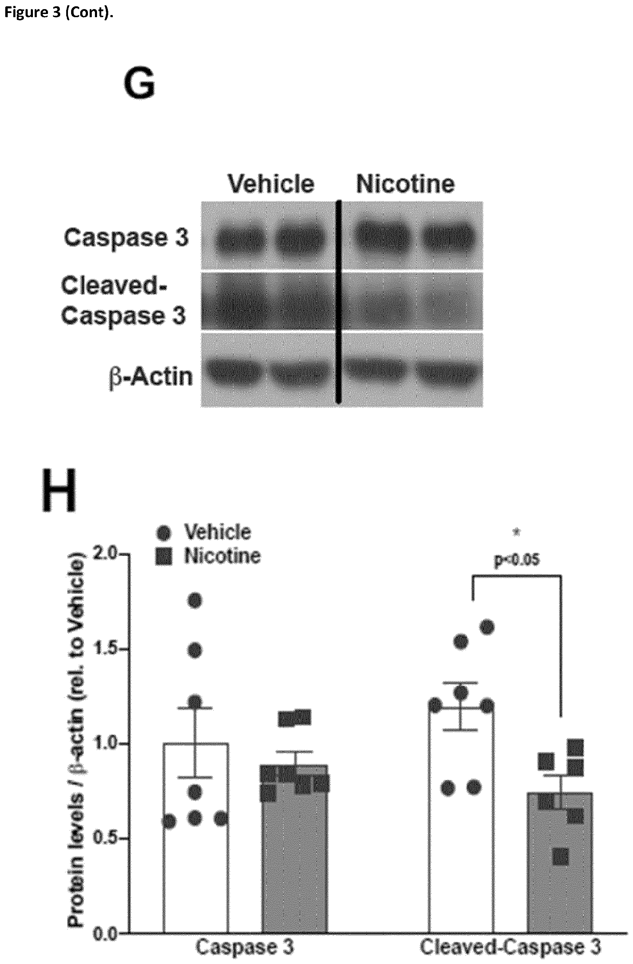

**Fig. 3****: Nicotine effect on brain AMPK in STD rats after cerebral ischemia**. A and C) Representative autoradiographic images; and B and D) protein levels quantification of the AMPK pathway in the STD rats treated and non-treated (vehicle) with nicotine. E) Representative autoradiographic images; and F) protein levels quantification of the ER stress pathway in the STD rats treated and non-treated (vehicle) with nicotine, respectively. G) Representative autoradiographic images; and H) protein levels quantification of Caspase-3 activation. All protein samples were obtained at 28 days after tMCAO from the ipsilateral hemisphere. The protein levels have been calculated as % concerning the STD non-treated rats and have been normalized by the levels of β-actin. Data are shown as mean±SEM. * p<0.05, **p<0.001, ***p<0.0001 compared to the vehicle STD group, using the statistical t-Student test (n=6 for each group).

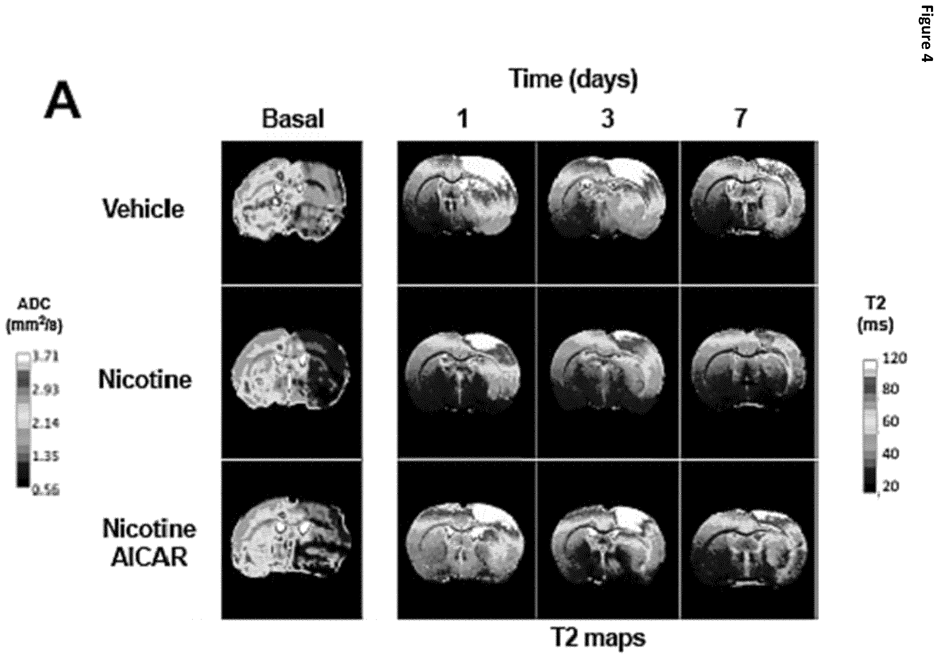

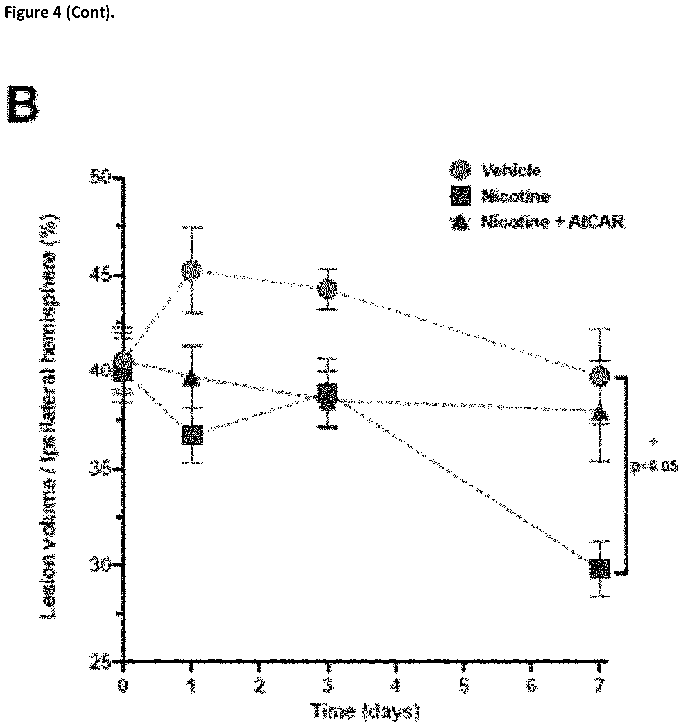

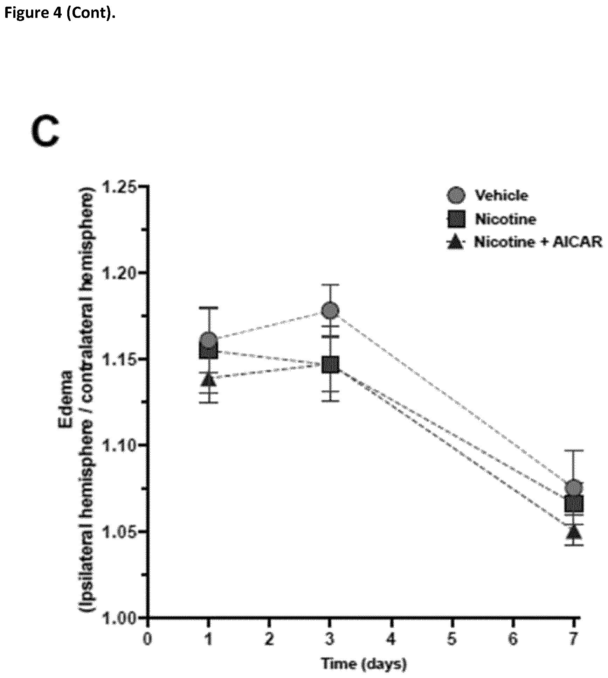

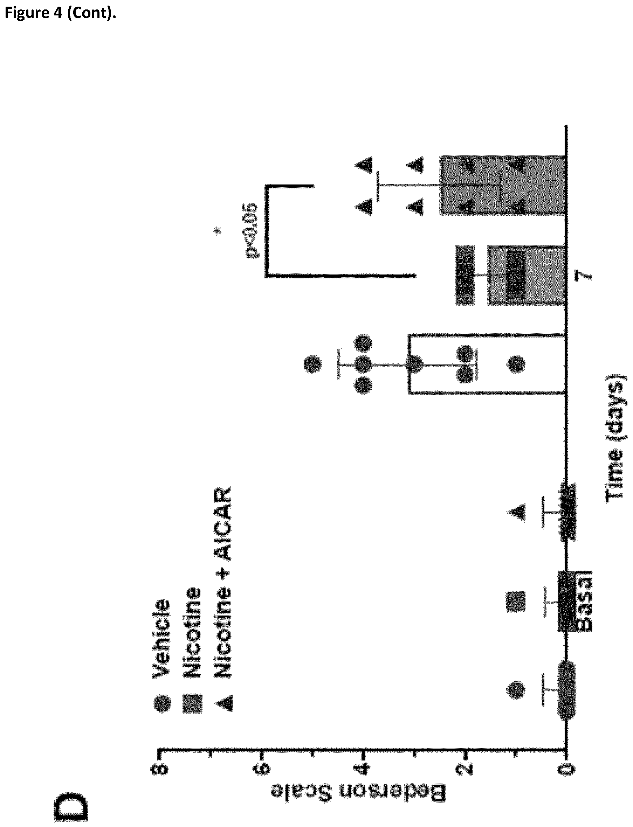

**Fig. 4****: Effect of CMV-AMPKa2-DN EVs on the neuroprotective action of nicotine.** A) Ischemic lesion and edema volumes were assessed by means of MRI. Representative ADC maps (which were obtained from DWI) showing initial homogeneous lesion volumes between STD groups treated and non-treated (vehicle) with nicotine, or AICAR. T2 relaxation maps (which were obtained from T2-weighted images) on each row correspond to one of 2D slices of the same animals within each group at days 1, 3 and 7 after ischemia onset. B and C) Time course of lesion and edema volumes. D) Sensorimotor deficits after ischemic insult were assessed using the Bederson Scale. Data are shown as mean±SEM. *p<0.05 compared to the vehicle group; using the two-way analysis of variance (ANOVA) statistical test; (n=6 for each group).

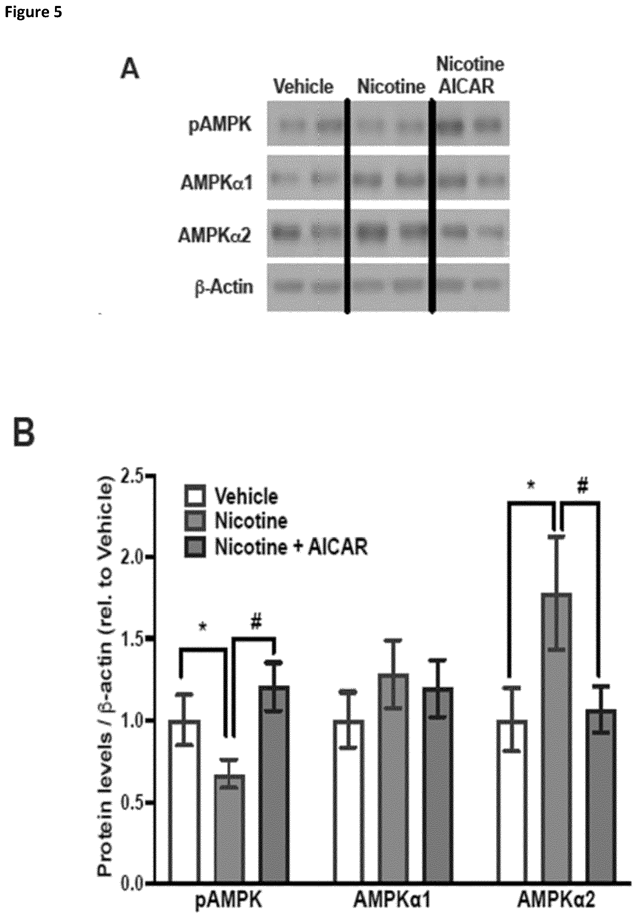

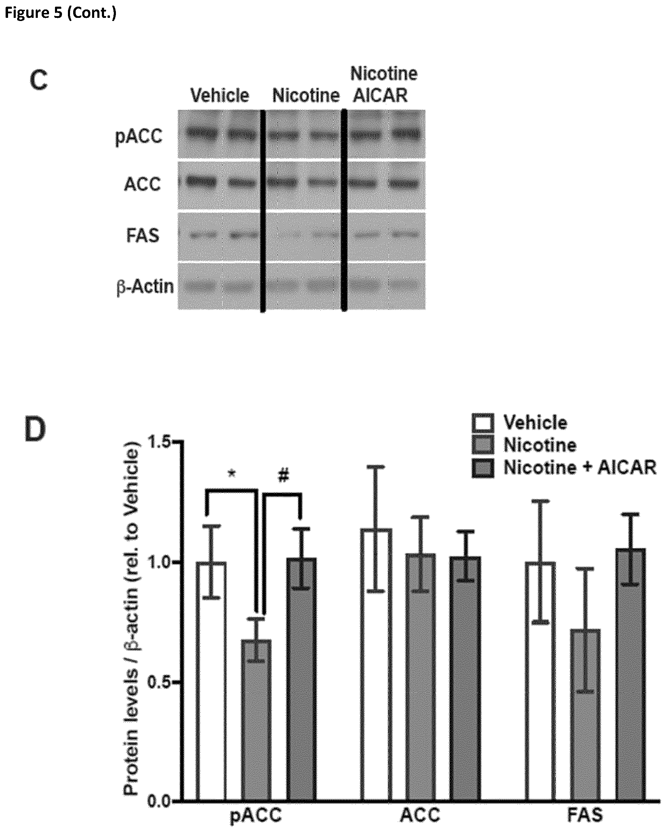

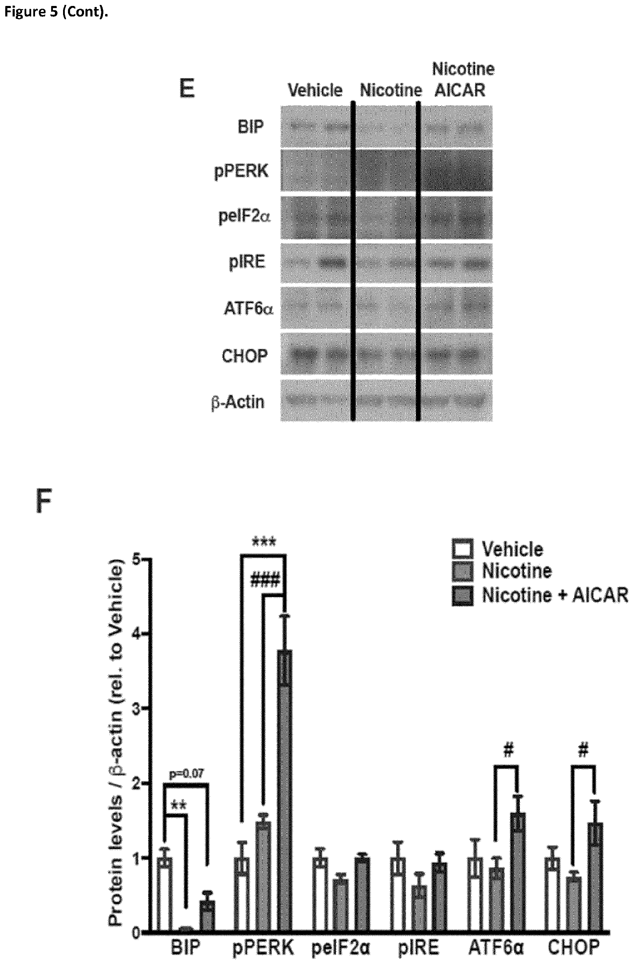

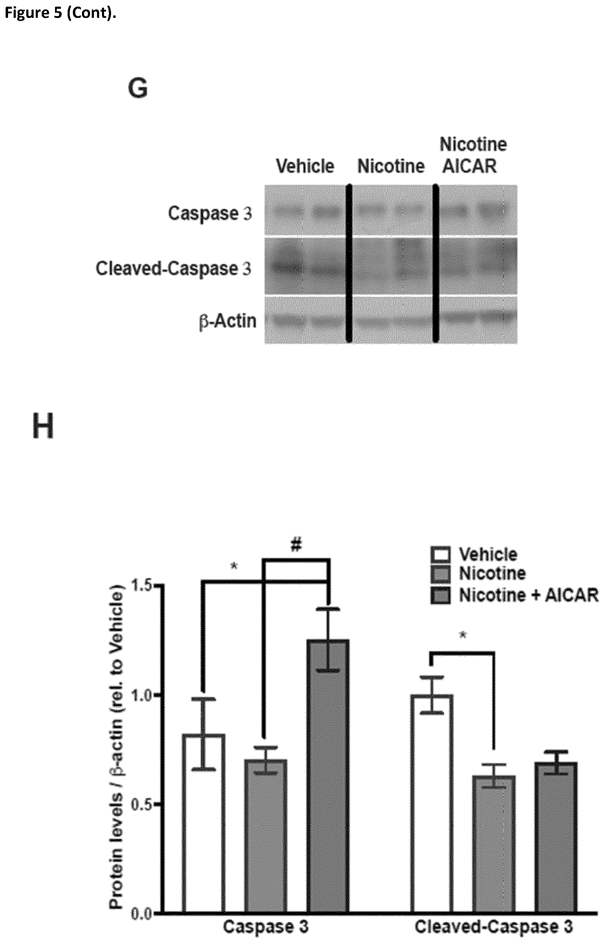

**Fig. 5****: Effect of AICAR on nicotine's action on AMPK signaling, ER stress, and Caspase-3 expression.** A and C) Representative autoradiographic images; and B and D) protein levels quantification of the AMPK pathway in STD ischemic rats treated and non-treated (vehicle) with nicotine, or nicotine+AICAR. E) Representative autoradiographic images; and F) protein levels quantification of the ER stress pathway in STD ischemic rats treated and non-treated (vehicle) with nicotine, or nicotine+AICAR. G) Representative autoradiographic images; and H) protein levels quantification of Caspase-3 activation. All the protein samples were obtained at 7 days after tMCAO from the ipsilateral hemisphere. The protein levels have been calculated as % with respect to the vehicle group and have been normalized by the levels of β-actin. Data are shown as mean±SEM. *p<0.05, **P<0.001, ***p<0.0001; #p<0.05, ##p<0.001, ###p<0.0001 compared to the vehicle group or nicotine group, respectively; using the two-way analysis of variance (ANOVA) statistical test; (n=6 for each group).

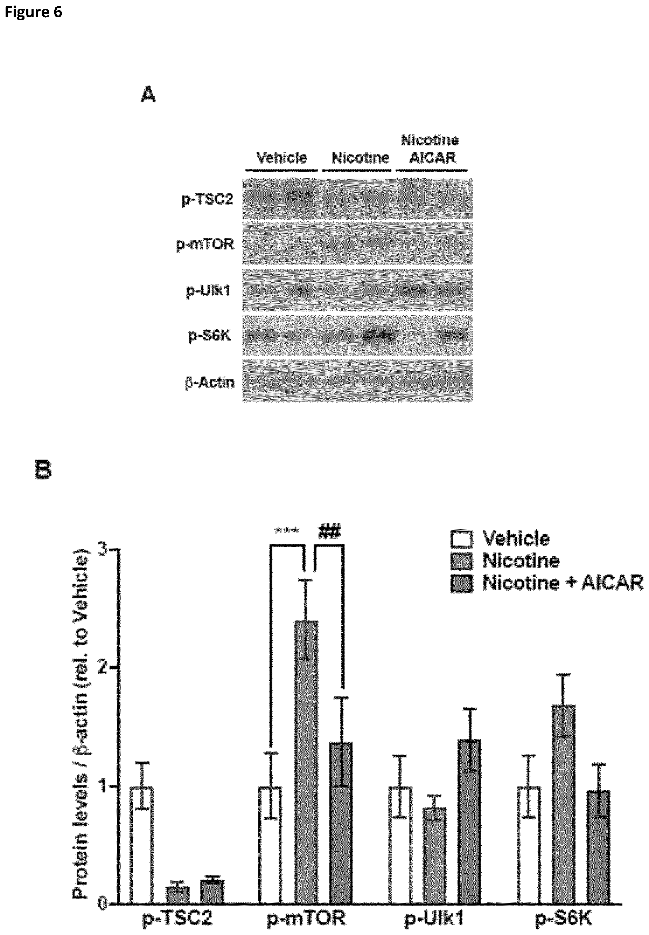

**Fig. 6****: Effect of AICAR on mTOR pathway.** A) Representative autoradiographic images; and B) protein levels quantification of the AMPK pathway in STD ischemic rats treated and non-treated (vehicle) with nicotine, or nicotine+AICAR. All the protein samples were obtained at 7 days after tMCAO from the ipsilateral hemisphere. The protein levels have been calculated as % concerning the vehicle group and have been normalized by the levels of β-actin. Data are shown as mean±SEM. ***p<0.0001; ##p<0.001, compared to the vehicle group or nicotine group, respectively; using the two-way analysis of variance (ANOVA) statistical test; (n=6 for each group).

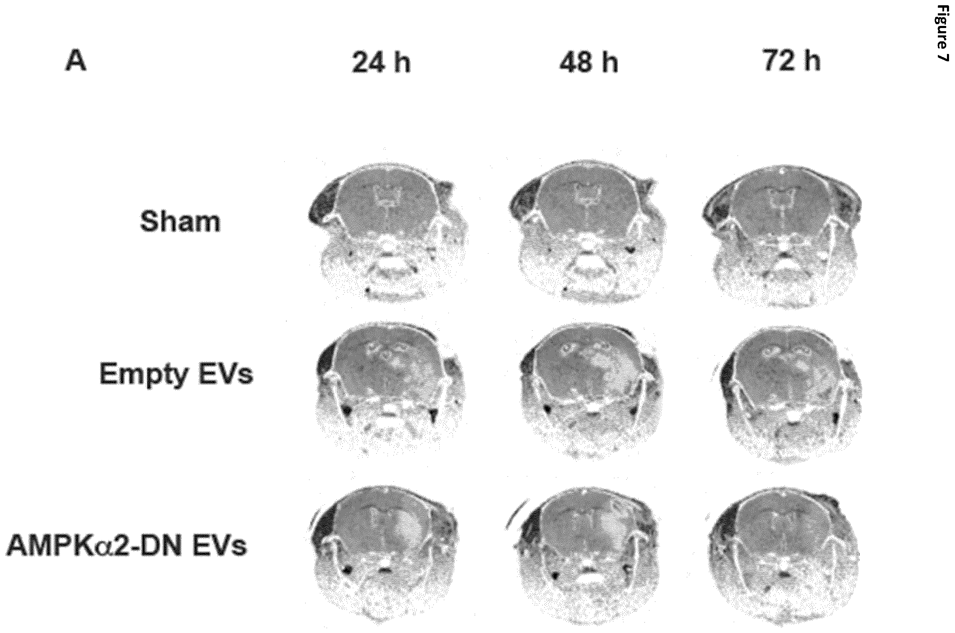

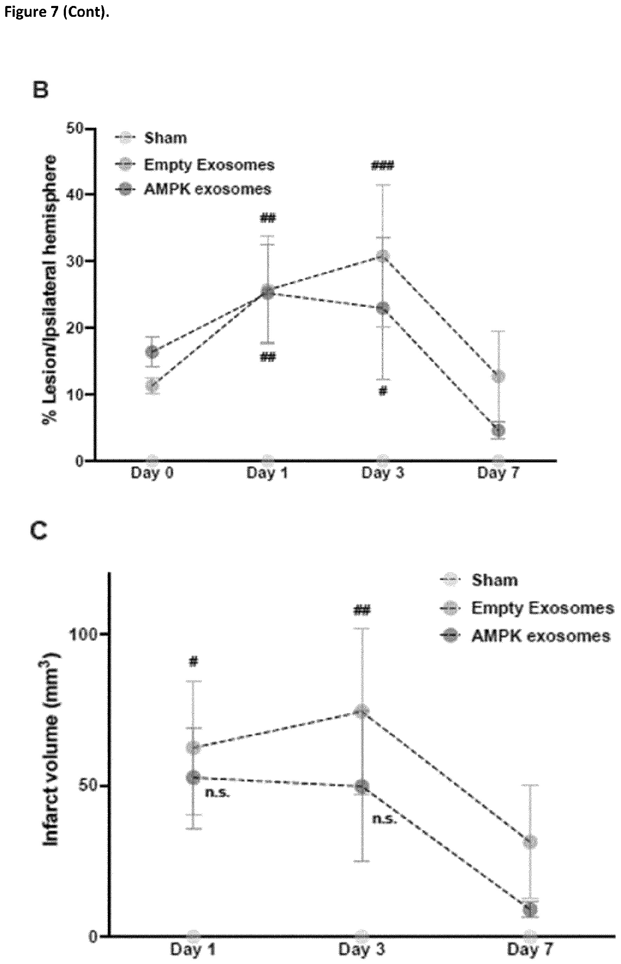

**Fig. 7****: Effect of CMV-AMPKa2-DN EVs on the ischemic.** A) Ischemic lesion and edema volumes were assessed by means of MRI. T2 relaxation maps (which were obtained from T2-weighted images) on each row correspond to one of 2D slices of the same animals within each group at days 1, 3 and 7 after ischemia onset. B and C) Time course of lesion and edema volumes. Data are shown as mean±SEM. #p<0.05, ##p<0.001, ###p<0.0001 compared to the Sham group; using the two-way analysis of variance (ANOVA) statistical test; (n=6 for each group).

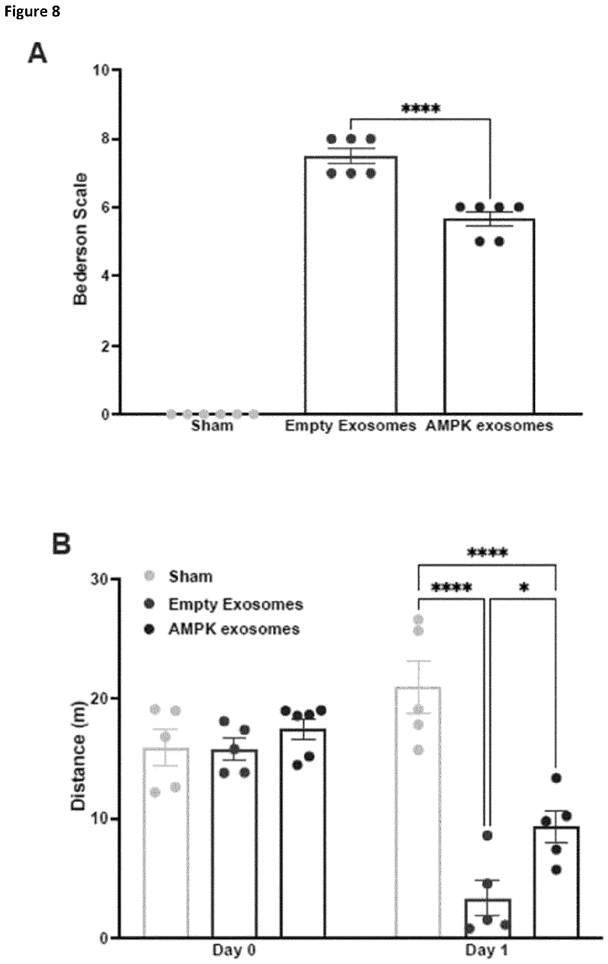

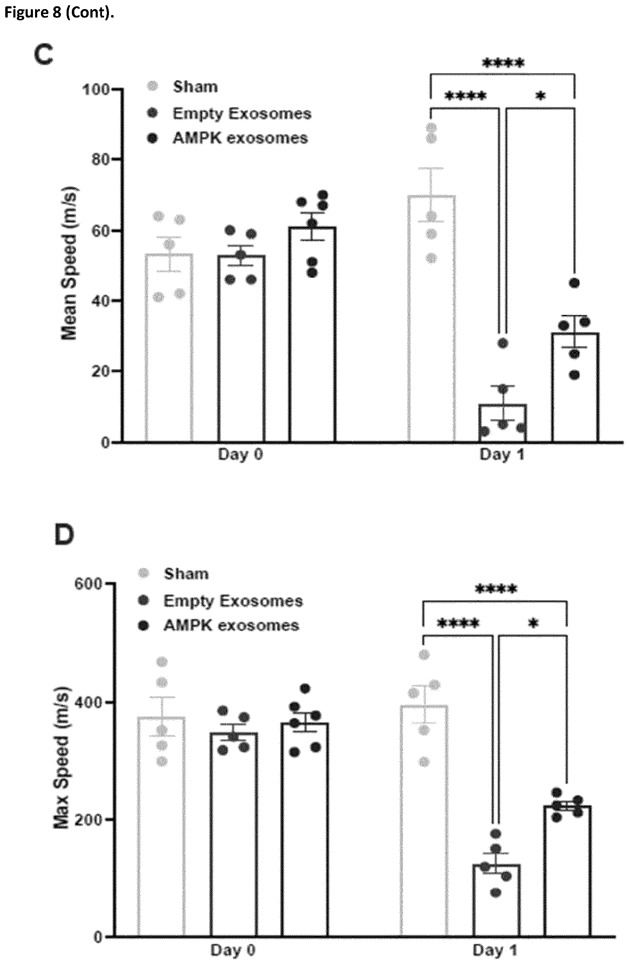

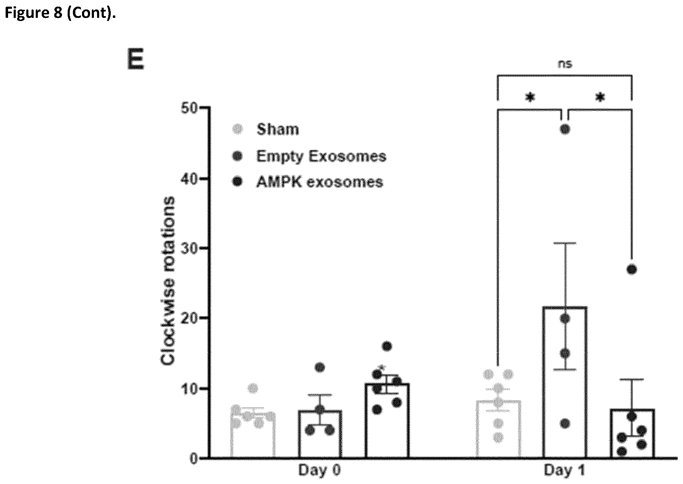

**Fig. 8****: Effect of CMV-AMPKa2-DN EVs on the neuroprotection after cerebral ischemia.** A) Sensorimotor deficits after ischemic insult were assessed using the Bederson Scale on day 1. Open Field test was carried out to analyze locomotor deficit in all groups, to determine B) travelled distance, C) average speed, D) maximum speed, E) rotations about the same body axis. Data are shown as mean±SEM. *p<0.05, **p<0.001, ***p<0.0001 compared to each group; using the two-way analysis of variance (ANOVA) statistical test; (n=6 for each group).

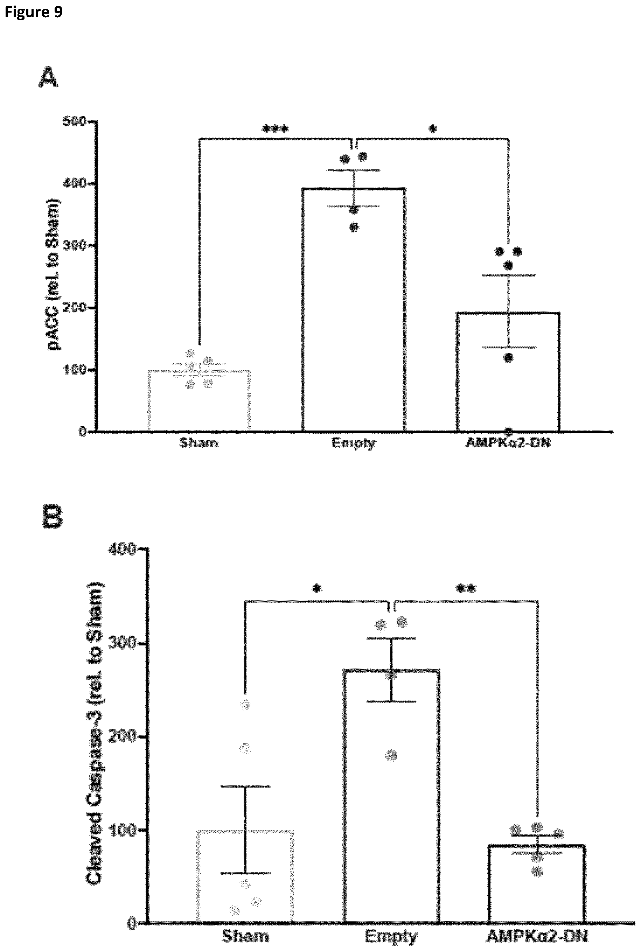

**Fig. 9****: Effect of CMV-AMPKa2-DN EVs on AMPK and apoptotic pathways.** A) Protein levels quantification of the AMPK pathway in mice treated with Empty EVs (Empty) or with CMV-AMPKa2-DN EVs (AMPKa2-DN), and Sham group. B) Protein levels quantification of activated Caspase-3 in mice treated with Empty EVs (Empty) or with CMV-AMPKa2-DN EVs (AMPKa2-DN), and Sham group. All the protein samples were obtained at 7 days after tMCAO from the ipsilateral hemisphere. The protein levels have been calculated as % concerning the Sham group and have been normalized by the levels of β-actin. Data are shown as mean±SEM. *p<0.05, **p<0.001, ***p<0.0001 compared to each group; using the two-way analysis of variance (ANOVA) statistical test; (n=6 for each group).

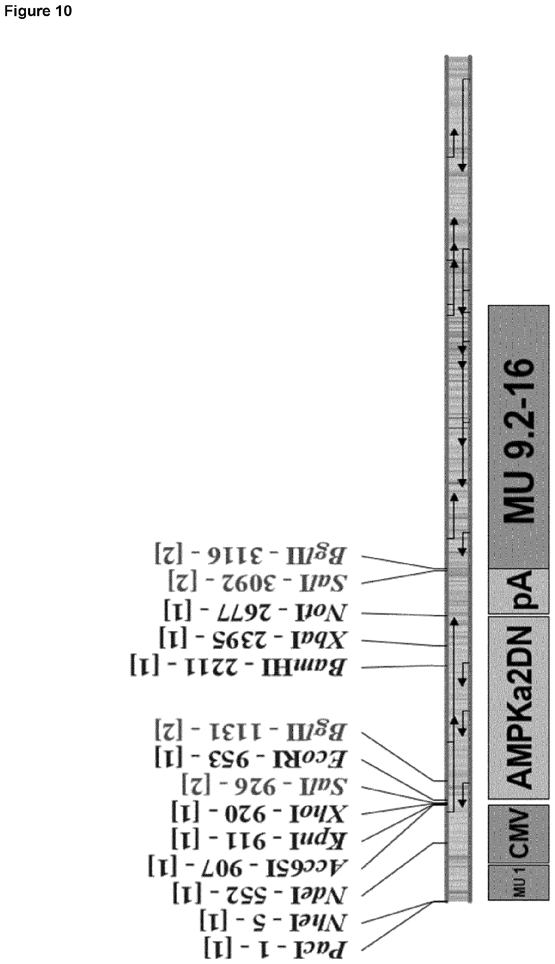

**Fig. 10****: Schematic representation of VQAd CMV AMPKa2DN 31487**

### DEFINITIONS

As used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

If the term "about" as used in connection with a numerical value throughout the specification and the claims denotes an interval of accuracy, familiar and acceptable to a person skilled in the art. For instance, the term "about" means the indicated value ± 1% of its value, or the term "about" means the indicated value ± 2% of its value, or the term "about" means the indicated value ± 5% of its value, the term "about" means the indicated value ± 10% of its value, or the term "about" means the indicated value ± 20% of its value, or the term "about" means the indicated value ± 30% of its value; preferably the term "about" means exactly the indicated value (± 0%).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of' excludes any element, step, or ingredient not specified in the claim element. When used herein, "consisting essentially of does not exclude materials or steps that do not materially affect the basic and novel characteristics of the claim.

As used herein, the term "extracellular vesicles" (EVs), refers to lipid bilayer-bound vesicles released from living cells into the extracellular environment. These vesicles lack functional nuclei and cannot replicate. Traditionally, EVs can be roughly classified into two main subtypes regarding their characteristics and biogenesis pathway: ectosomes and small extracellular vesicles (sEVs). Ectosomes have a size distribution between 100 and 1000 nm and are generated by cytoplasmic membrane budding. sEVs, where exosomes are included, are smaller in diameter (usually between 30-150 nm) and released by the fusion of the multivesicular bodies (MVBs) with the plasma membrane. The components of sEVs indicate their cellular origin and potential biological functions. In the context of the present invention, it is preferred that the sEVs or exosomes are produced in antigen-presenting cells, as is explained in detail below.

"Nucleic acids," "nucleic acid molecules," "oligonucleotide," and "polynucleotide" are used interchangeably and refer to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix.

As used herein, the term "gene" or "coding sequence" refers to a polynucleotide sequence in vitro or in vivo that encodes a gene product. In some instances, the gene consists or consists essentially of the coding sequence, that is, a sequence that encodes the gene product.

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulphur containing reducing agents, such as urea, glutathione, thioctic acid, sodium thioglycolate, thioglycerol, [alpha]-monothioglycerol, and sodium thio sulphate; low molecular weight proteins, such as human serum albumin, bovine serum albumin, gelatin, or other immunoglobulins; and hydrophilic polymers, such as polyvinylpyrrolidone.

The terms "sequence identity" or "percent identity" in the context of two or more nucleotide sequences, polypeptide sequences or proteins sequences refer to two or more sequences or subsequences that are the same ("identical") or have a specified percentage of nucleotide or amino acid residues that are identical ("percent identity") when compared and aligned for maximum correspondence with a second molecule, as measured using a sequence comparison algorithm, preferably BLAST alignment tool, or alternatively, by visual inspection. The "sequence identity" or "percent identity" can be determined by calculating the number of identical nucleotides or amino acids at the same positions in a nucleic acid, polypeptide or protein. Calculation of percent identity includes determination of the optimal alignment between two or more sequences. Alignment can take into account insertions and deletions (i.e. "gaps") in each of the sequences to be tested, such as, without limitation, in the non-coding regions of nucleic acids and truncations or extensions of polypeptide sequences. Computer programs and algorithms such as the Basic Local Alignment Search Tool (BLAST) may be used to determine the percent identity. BLAST is one of the many resources provided by the U.S. National Center for Biotechnology Information. Preferably, the "percentage of identity" as used herein is decided in the context of a local alignment, i.e., it is based on the alignment of regions of local similarity between nucleobase sequences, contrary to a global alignment, which aims to align two sequences across their entire span. Thus, in the context of the present invention, percentage identity is calculated preferably only based on the local alignment comparison algorithm. Also, because the genetic code is degenerate, and more than one codon can encode a given amino acid, coding regions of nucleic acids are considered identical if the nucleic acids encode identical polypeptides. Thus, percent identity could also be calculated based on the polypeptide encoded by the nucleic acid. Percent identity could be calculated based on full length consensus genomic sequences or on a fraction of the genomic sequence, such as for example without limitation on individual open reading frames (ORFs).

"Percent (%) amino acid sequence identity" with respect to proteins or polypeptides described herein is defined as the percentage of amino acid residues in a candidate sequence that are identical with the amino acid residues in the reference sequence (i.e., the protein or polypeptide from which it is derived), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for example, using publicly available computer software such as BLAST. Those skilled in the art can determine appropriate parameters for measuring alignment, including any algorithms needed to achieve maximum alignment over the full-length of the sequences being compared. The similar applies to "Percent (%) nucleotide sequence identity", but for nucleic acids.

Numeric ranges are inclusive of the numbers defining the range.

As used herein, the term "AMP-activated protein kinase" (AMPK) refers to an enzyme that plays a role in cellular energy homeostasis, largely to activate glucose and fatty acid uptake and oxidation when cellular energy is low. It belongs to a highly conserved eukaryotic protein family. It consists of three proteins (subunits) that together make a functional enzyme, conserved from yeast to humans. It is expressed in a number of tissues, including the liver, brain, and skeletal muscle. In response to binding AMP and ADP, the net effect of AMPK activation is stimulation of hepatic fatty acid oxidation, ketogenesis, stimulation of skeletal muscle fatty acid oxidation and glucose uptake, inhibition of cholesterol synthesis, fatty acid synthesis, and triglyceride synthesis, inhibition of adipocyte lipogenesis, , and modulation of insulin secretion by pancreatic beta-cells. AMPK is a heterotrimeric protein complex that is formed by α, β, and γ subunits. As used herein, "AMPKα" refers to the subunit alpha (α) of the AMPK protein. The α, β, and γ subunits can also be found in different isoforms: the γ subunit can exist as either the γ1, γ2 or γ3 isoform; the β subunit can exist as either the β1 or β2 isoform; and the α subunit can exist as either the α1 or α2 isoform.

### DESCRIPTION

In **a first aspect**, the present invention relates to a population of small extracellular vesicles (sEVs), also called herein the "sEVs of the invention", comprising at least one polynucleotide comprising a gene encoding a dominant negative AMP-activated protein kinase alpha 2 (AMPKα2-DN) mutant protein. Preferably, the sEVs are exosomes, also called herein the "exosomes of the invention".

As used herein, "AMPKα2" refers to the isoform 2 of the subunit α of AMPK protein, preferably the AMPK protein from *mus musculus.* The term "dominant negative AMPKα2 mutant protein" (AMPKα2-DN) refers to an inactive isoform of the AMPKα2. Preferably, the inactive isoform of AMPKα2 is a AMPKα2 protein modified in its active site with at least the point mutation D157A, wherein the amino acid position (157) is expressed with respect to the wild-type AMPKα2 sequence, preferably the wild-type *mus musculus* AMPKα2 sequence of SEQ ID NO: 1 or SEQ ID NO: 8.

However, although the *mus musculus* sequence is the preferred sequence, the sequence of the AMPKα2-DN protein can be the sequence of a homologous sequence of the *mus musculus* AMPKα2 protein corresponding to the species where the treatment is applied, such as human homologous of AMPKα2 protein, or humanised or a codon-optimized AMPKα2 protein for treatment in humans.

Please note that here and thorough the whole document, the positions or locations of the amino acid residues of a protein or a polypeptide sequence are numbered sequentially starting from the first amino acid residue which would then be located at position 1. For example, a protein of 137 amino acids will have those residues numbered 1 (first amino acid residue) until 137 (the last amino acid residue). Preferably, the first position or position number 1 corresponds to the first amino acid residue located at the 5-prime (5') end of the polypeptide chain that has a nitrogen atom or a free amino group. Thus, the numbering preferably starts from the first amino acid residue at the N terminal or 5' end of the protein or polypeptide and ends at the 3' end or C terminal end of the protein or polypeptide. Preferably, the positions of the amino acid residues are numbered using the amino acid sequence of the translated mature protein. It is also noted that the amino acid numbering, and therefore the number of the amino acid substitution that results in AMPKα2-DN mutant protein as defined herein, may change within AMPKα2 proteins from different species.

A single nucleotide substitution that results in a codon change encoding a different amino acid is called "missense point mutation" (called "mutation" or "substitution" thereafter). In the context of the present invention, missense point mutations or substitutions are noted following the structure "A1-number-A2", wherein "A1" is the amino acid residue that is replaced, "number" is the amino acid position within the sequence where A1 is, and "A2" is the amino acid that is used instead of "A1". For example, a substitution D157A in SEQ ID NO:1, as used herein, means that the amino acid aspartic acid in position number 157 in SEQ ID NO: 1 is substituted by an alanine.

In an embodiment, the AMPKα2-DN mutant protein encoded by the at least one polynucleotide comprised in the sEVs or exosomes comprises, consists, or consists essentially of an amino acid sequence with at least 50%, 60%, 70%, 75%, 80%, 85%, 90%, 91%, 92%, 93%, 94%, 95%, 96%, 97%, 98%, or 100% sequence identity with SEQ ID NO: 1, with the proviso that the amino acid residue in position number 157 in SEQ ID NO: 1, which corresponds with an aspartic acid, is substituted by an amino acid residue that is not aspartic acid. Preferably, the aspartic acid residue in position number 157 in SEQ ID NO: 1 is substituted by an alanine (mutation D157A). Preferably, the AMPKα2-DN mutant protein comprises a sequence that is at least 85%, 90%, preferably 95% identical to SEQ ID NO: 1, with the proviso that the amino acid residue in position number 157 in SEQ ID NO: 1, which corresponds with an aspartic acid, is substituted by an amino acid residue that is not aspartic acid, preferably alanine. Preferably, the AMPKα2-DN mutant protein is SEQ ID NO: 1, with the proviso that the amino acid residue in position number 157 in SEQ ID NO: 1, which corresponds with an aspartic acid, is substituted by an amino acid residue that is not aspartic acid, preferably alanine.

In an embodiment, the AMPKα2-DN mutant protein encoded by the at least one polynucleotide comprised in the sEVs or exosomes comprises, consists, or consists essentially of an amino acid sequence with at least 50%, 60%, 70%, 75%, 80%, 85%, 90%, 91%, 92%, 93%, 94%, 95%, 96%, 97%, 98%, or 100% sequence identity with SEQ ID NO: 2, with the proviso that the amino acid residue in position number 169 in SEQ ID NO: 2 is not an aspartic acid. In an embodiment, the AMPKα2-DN mutant protein encoded by the at least one polynucleotide comprised in the sEVs or exosomes comprises, consists, or consists essentially of an amino acid sequence with at least 50%, 60%, 70%, 75%, 80%, 85%, 90%, 91%, 92%, 93%, 94%, 95%, 96%, 97%, 98%, or 100% sequence identity with SEQ ID NO: 2, with the proviso that the amino acid residue in position number 169 in SEQ ID NO: 2 corresponds to amino acid alanine (169A).

In an embodiment, the AMPKα2-DN mutant protein encoded by the at least one polynucleotide comprised in the sEVs or exosomes comprises, consists, or consists essentially of an amino acid sequence with at least 85%, 90%, preferably 95%, sequence identity with SEQ ID NO: 2, with the proviso that the amino acid residue in position number 169 in SEQ ID NO: 2 is not an aspartic acid. Preferably, the AMPKα2-DN mutant protein comprises or consists of SEQ ID NO: 2.

In an embodiment, the gene encoding for the AMPKα2-DN mutant protein comprises, consists, or consists essentially of polynucleotide sequence with at least 50%, 60%, 70%, 75%, 80%, 85%, 90%, 91%, 92%, 93%, 94%, 95%, 96%, 97%, 98%, or 100% sequence identity with SEQ ID NO: 3. Preferably, the gene encoding for the AMPKα2-DN mutant protein comprises or consists of SEQ ID NO: 3.

In an embodiment, the gene encoding for the AMPKα2-DN protein is under the control of a promoter, that is also comprised in the polynucleotide. A coding sequence and a gene expression control sequence or promoter are said to be operably linked when they are linked in such a way as to place the expression or transcription and/or translation of the coding sequence under the influence or control of the gene expression control sequence. For example, the gene encoding the AMPKα2-DN may be operably linked to the CMV promoter so that the expression levels of the AMPKα2-DN are regulated by the CMV promoter.

In an embodiment, the promoter is a ubiquitous or a tissue specific promoter. As used herein, a "tissue specific" promoter is a promoter that is only active in specific types of cells/tissues, preferably specific mammal tissues, most preferably specific human tissues. As used herein, a ubiquitous promoter is a promoter that is active in many or in any different tissue(s), preferably mammal tissues, most preferably human tissues. Usually, "many" in this context may mean more than 5 or at least 6, 10, 15, 20 or in 5, 6, 7, 8, 9, 10, 11, 12, 13, 14, 15, 16, 17, 18, 19, 20 different tissues. In a preferred embodiment, the promoter used to regulate the expression of the AMPKα2-DN protein is ubiquitous and/or constitutive. In a more preferred embodiment, said promoter is ubiquitous and constitutive. A "constitutive" promoter is a promoter that is active under most physiological and developmental conditions. Preferably, the promoter is selected from the list consisting of cytomegalovirus promoter (CMV), SV40, CMV, UBC, EF1A, PGK and CAGG. Preferably, the ubiquitous and constitutive promoter that drives the expression of AMPKα2-DN protein in the sEVs or exosomes of the invention is the CMV promoter. In an embodiment, the CMV promoter comprises, consists, or consists essentially of a sequence with at least 50%, 60%, 70%, 75%, 80%, 85%, 90%, 91%, 92%, 93%, 94%, 95%, 96%, 97%, 98%, or 100% sequence identity with SEQ ID NO: 4. Preferably, the CMV promoter comprises or consists of SEQ ID NO: 4.

In another embodiment, the promoter used to regulate the expression of the AMPKα2-DN protein is ubiquitous and/or inducible. An "inducible" promoter is a promoter that is preferably regulated depending on physiological or developmental conditions, preferably in physiological or developmental conditions of hypoxia. In a more preferred embodiment, said promoter is ubiquitous and inducible in hypoxia conditions. Preferably, the ubiquitous and inducible promoter that drives the expression of AMPKα2-DN protein in the sEVs or exosomes of the invention, under hypoxic conditions, is heat-shock protein 70 (Hsp70) promoter, also known as Hspa1a or Hsp68. Hsp70 is a transcription factor, which expression was found to be increased under hypoxia. Preferably, the promoter that drives the expression of AMPKα2-DN protein in the sEVs or exosomes of the invention is the heat-shock protein 70 (Hsp70) promoter. In an embodiment, the Hsp70 promoter comprises, consists, or consists essentially of a sequence with at least 50%, 60%, 70%, 75%, 80%, 85%, 90%, 91%, 92%, 93%, 94%, 95%, 96%, 97%, 98%, or 100% sequence identity with SEQ ID NO: 9. Preferably, the Hsp70 promoter comprises or consists of SEQ ID NO: 9.

Additionally, the Hypoxia-Inducible Factor 1 (HIF-1) is a crucial transcription factor involved in the cellular response to hypoxia. In normal concentrations, HIF-1 is rapidly degraded, however, in hypoxic conditions its structure is stabilized, binding to specific regions of certain promoters called hypoxia-responsive elements (HRE), such as its own or VEGF among others to increase your expression. The consensus sequence for HRE is 5'-ACGTGNNNNACGTG-3' (SEQ ID NO: 7), which can be added to the 5' end of the promoter (e.g., CMV or Hsp70 promoter) to enhance the expression under hypoxic conditions. Preferably, the promoter that drives the expression of AMPKα2-DN protein in the sEVs or exosomes of the invention is an CMV promoter comprising the HRE sequence in its 5' end. Preferably, the promoter that drives the expression of AMPKα2-DN protein in the sEVs or exosomes of the invention is an Hsp70 promoter comprising the HRE sequence in its 5' end.

Hence, the population of sEVs or exosomes provided herein comprise or are loaded with at least one polynucleotide comprising a gene encoding an AMPKα2-DN mutant protein. Preferably, each of the sEVs of the population is loaded with at least one polynucleotide comprising a gene encoding an AMPKα2-DN mutant protein, wherein said gene may be operably linked and under the control of a CMV promoter. Preferably, at least one polynucleotide is DNA, more preferably a plasmid. Thus, designing a plasmid encoding for AMPKα2-DN protein under the control of CMV promoter (CMV-AMPKα2-DN plasmid) will allow the AMPKα2-DN to be expressed in most, if not all tissues. Once the sEVs or exosomes are fused with the target cells, the AMPKα2-DN mutant protein would be expressed in the target cell. The techniques used to load sEVs with various cargo include free-thaw cycles to fuse sEVs and liposomes, sonication, extrusion, permeabilization with saponin, and electroporation. Several commercial kits are available for loading nucleic acids into sEVs and the skilled person would be familiar with them. Preferably, the at least one polynucleotide, preferably a plasmid, is mainly located in the core of the sEVs or exosomes.

The sEVs or exosomes of the invention may further comprise or be loaded with, preferably mainly at their outer membrane, at least a fusion protein. Said fusion protein, also named Lamp2b-RVG protein, comprises or consists of the neurotrophic rabies virus (RVG) peptide fused to lysosome-associated membrane protein 2b (Lamp2b). Preferably, said fusion protein comprises, consists or consists essentially of an amino acid sequence with at least 50%, 60%, 70%, 75%, 80%, 85%, 90%, 91%, 92%, 93%, 94%, 95%, 96%, 97%, 98%, or 100% sequence identity with SEQ ID NO: 5. Preferably, the fusion protein comprises or consists of SEQ ID NO: 5. By "fusion protein" is referred herein as to a protein formed by at least two domains, wherein the at least two domains have been joined, one after the other, so that they are synthetized or translated as a single unit, and thus the two domains of the fusion protein are part of a single polypeptide. In this particular case, the domains comprised or consisting of the fusion protein are the RVG peptide and the Lamp2b protein. In an embodiment, the domains of the Lamp2b-RVG fusion protein may be linked by a linker peptide. "Linker peptide" as used herein is a short peptide sequence that is located between the domains of a fusion protein. Linker peptides are placed to provide the two domains comprised in the fusion protein with movement flexibility. In the context of the present invention, the linker peptide has at least one amino acid residue, preferably at least two consecutive amino acid residues, optionally 3, 4, 5, 6, 7, 8, 9, 10, 11, 12, 13, 14, 15, 16, 17, 18, 19, 20 or 20 more amino acid residues. The linker peptide includes flexible linkers, rigid linkers, and in vivo cleavable linkers.

Therefore, it is preferred that the sEVs or exosomes of the invention comprise at least one polynucleotide encoding an AMPKα2-DN protein and are produced to have in their outer membrane at least one fusion protein, said fusion protein comprising the neurotrophic rabies virus (RVG) peptide fused to lysosome-associated membrane protein 2b (Lamp2b). For that purpose, a plasmid comprising the Lamp2b-RVG gene can be transfected in exosome generating-cells, preferably in exosome generating-immature dendritic cells, to transiently express the Lamp2b-RVG fusion protein comprising the neurotrophic rabies virus (RVG) peptide fused to lysosome-associated membrane protein 2b, wherein, preferably, such fusion protein comprises an amino acid sequence with at least 95% sequence identity with SEQ ID NO 5. This way, when the sEVs or exosomes are produced, they will carry the Lamp2b-RVG fusion protein.

Preferably, the sEVs or exosomes of the invention comprise at least one polynucleotide comprising a gene encoding an AMPKα2-DN mutant protein operably linked and under the control of a ubiquitous promoter, preferably CMV, and comprise in their outer membrane at least one fusion protein comprising the RVG peptide fused to Lamp2b. Preferably, the sEVs or exosomes of the invention comprise at least one polynucleotide comprising a gene encoding an AMPKα2-DN mutant protein operably linked and under the control of the HSp70 promoter, and comprise in their outer membrane at least one fusion protein comprising the RVG peptide fused to Lamp2b.

Preferably, the sEVs or exosomes of the invention comprise at least one polynucleotide encoding AMPKα2-DN mutant protein operably linked and under the control of a tissue-specific promoter and comprise in their outer membrane at least one fusion protein comprising the RVG peptide fused to Lamp2b.

In an embodiment, the polynucleotide comprised in the sEVs of the invention, preferably in the exosomes of the invention, comprises, consists, or consists essentially of a sequence with at least 50%, 60%, 70%, 75%, 80%, 85%, 90%, 91%, 92%, 93%, 94%, 95%, 96%, 97%, 98%, or 100% sequence identity with SEQ ID NO: 6. Preferably, the polynucleitde comprises or consists of SEQ ID NO: 6.

In an embodiment, the sEVs or exosomes further comprise markers derived from the cells used to produce them (hereinafter referred to as producer cell). Preferably, the producer cells are immune cells, more preferably antigen presenting cells, and even more preferably dendritic cells. Preferably, the producer cells are mammalian cells. The producer cell may be a primary cell or an immortalized cell. In an embodiment, the producer cell is a cell with low immunogenicity, preferably an immature immune cell, preferably an immature antigen presenting cell, and most preferably an immature dendritic cell or monocyte. By "immature" is referred herein as a cell that is not activated or biologically active, or that does not present activation markers or molecules on the surface. An immature dendritic cell has a different morphological phenotype than the mature one. Immature dendritic cells have a round and smooth surface, while mature cells, such as mature dendritic cells, have a rough surface with multiple pseudopodia. Immature dendritic cells produce large quantities of exosomes devoid of T-cell activators such as MHC-II, CD80 and CD86. Thus, in an embodiment, the producer cell, preferably the immature dendritic cell, does not express activation markers, preferably T-cell activator markers or molecules such as major histocompatibility complex II (MHC-II), cluster of differentiation 80 (CD80) or cluster of differentiation 86 (CD86), or a combination thereof. In an embodiment, the producer cell is an immature immortalized cell, preferably an immature immortalized dendritic cell or monocyte, most preferably the JAWS II cell line from the American Type Culture Collection CRL-1194; ATCC; Manassas, VA, USA or a cell derived from said JAWS II cell line. Also, preferably, the producer cell is a genetically modified cell that expresses the RVG-Lamp2b fusion protein, preferably in its outer membrane. In an embodiment, the producer cell is an immature antigen presenting cell characterized by having a statistically significant reduced expression of at least 20%, 30%, 40%, 50%, 60%, 70%, 80%, 90%, 95% or 100% of at least one T-cell activator molecule in comparison to the expression of said T-cell activator molecule in a mature antigen presenting cell. The percentage of reduction of said T-cell activator molecule can be measured by techniques known in the art, e.g. by flow cytometry or RT-PCR.

The producer cells may be grown in a conditioned media, and the sEVs or the exosomes are produced and released to said media. In the context of the present invention, the term "conditioned media" is understood as the supernatant of cell cultures. Since the population of sEVs or exosomes is released into the extracellular media when the intracellular multivesicular bodies fuse with the plasma membrane of the producer cell, the sEVs or exosome outer membrane would be similar to the one of the producing cell. In particular embodiments, the producer cell is a mammalian cell, e.g., a human cell. In particular embodiments, the producer cell is an immature dendritic cell. Thus, in an embodiment, the population of sEVs or exosomes are positive for cellular markers, preferably cellular immature dendritic cell (iDC) markers. Further preferred, the population of sEVs or exosomes comprise one or more of the specific exosome markers selected from the groups consisting of ALIX, TSG101, CD9 or CD81, or any combination thereof. In another embodiment, the sEVs or exosomes are characterized by lacking the MHC-II, CD80, CD86 or GRP94 markers, or any combination thereof. In an embodiment, the sEVs or exosomes have an immature phenotype, so that they are low immunogenic.

In some embodiments, the sEVs or exosomes are produced from autologous producer cells of the subject to be treated or were obtained from cells that were isolated from the subject to be treated. In other embodiments, the sEVs or exosomes are produced from allogeneic producer cells to the subject to be treated or were obtained from cells that were isolated from a donor other than the subject to be treated. In particular embodiments, the producer cell is an autologous mammalian cell, e.g., an autologous human cell. In particular embodiments, the producer cell is an autologous immature dendritic cell.

Preferably, the sEVs or exosomes are isolated. In the context of the present invention, the term "isolated" indicates that the sEVs or exosomes or population thereof are not within the environment or cell culture where they were produced. The term isolated also encompasses a population of sEVs or exosomes that have been removed from the environment or ell culture, e.g., from the supernatant or conditioning media, from which they originated. In an embodiment, the population of sEVs or exosomes is a substantially pure population, and the sEVs or exosomes or population thereof has been substantially separated from surrounding environment or cell culture. In some embodiments, the population is substantially pure or enriched in sEVs or exosomes and comprises at least about 50%, 55%, 60%, 70%, 75%, 80%, 85%, 90%, 95%, 98%, or 100% of sEVs or exosomes.

In an embodiment, the population is pure in sEVs or exosomes. They are released by many cell types as a means of communicating with other cells and thus cell content may be potentially removed from the population. The cargo of extracellular vesicles includes the proteins, lipids, nucleic acids, and membrane receptors of the cells from which they originate.

Preferably, the population according to the first aspect or any of its embodiments comprises a substantially pure population of immature dendritic cells (iDC)-derived sEVs or exosomes, wherein the iDC-derived sEVs or exosomes, preferably each of the iDC-derived sEVs or exosomes, comprise at least one polynucleotide comprising a gene encoding an AMPKα2-DN mutant protein operably linked and under the control of a CMV promoter, and wherein the iDC-derived sEVs or iDC-derived exosomes have in their outer membrane at least one fusion protein comprising the RVG peptide fused to Lamp2b.

In an embodiment, the sEVs or exosomes are spherical or round-shaped. Further, the sEVs or exosomes may have a size of greater than 2 nm. The sEVs or exosomes may have a size of greater than 5 nm, 10 nm, 20 nm, 30 nm, 40 nm, 50 nm, 60 nm, 70 nm, 80 nm, 90 nm, 100 nm, 110 nm, 120 nm, 130 nm, 140 nm, 150 nm or 160 nm. The exosome may have a size of substantially 160 nm or greater. The sEVs or exosomes may have a range of size distribution, such as between 30 nm to 50 nm, 30 nm to 100 nm, preferably 30 nm to 150 nm or 30 nm to 200 nm. The size distribution may be determined by various means. In principle, the size may be determined by size fractionation and filtration through a membrane with the relevant size cut-off. The sEVs or exosomes size may then be determined by tracking segregation of component proteins with SDS-PAGE or by a biological assay. The size may also be determined by electron microscopy or by Nanoparticle tracking analysis (NTA).

As discussed above, the results provide evidence that the population of sEVs or exosomes described herein is able to decrease or reduce the activation of AMPKα2 in the target cell/tissue. In view of this, in a preferred embodiment, the population of sEVs or exosomes according to the first aspect or any of its embodiments is capable, preferably when administered systemically, of significantly decreasing the activation levels of AMPKα2 in the target cells in comparison to the activation levels of AMPKα2 in untreated cells or reference cells, preferably neurons. Thus, as a result of AMPKα2 inhibition, the exogenous AMPKα2-DN isoform lacks kinase activity and therefore the AMPK heterotrimer (alpha, beta, gamma; preferably alpha2 in this case) cannot be properly regulated and exert its phosphorylation effect on downstream targets. By "AMPKα2 inhibition" is referred herein as a decrease in AMPKα2 activation levels.

Preferably, the sEVs or exosomes of the invention are capable of reducing, preferably statistically significant reducing, the activation levels of AMPKα2 in the target cell, preferably in neurons. By "statistically significant" or "significant" is referred herein as the determination by an analyst that the results in the data are not explainable by chance alone. Statistical hypothesis testing is the method by which the skilled person makes this determination. This test provides a p-value, which is the probability of observing results as extreme as those in the data, assuming the results are truly due to chance alone. A p-value of 0.1 or lower (preferably 0.05, 0.01, 0.001 or lower) is considered herein to be statistically significant. For example, the reduction in the activation levels of AMPKα2 protein is a statistically significant reduction or decrease when a statistical test is performed to compare sEVs- or exosomes-treated target cells with sEVs- or exosomes-untreated target or reference cells, and wherein the resulting p-value of said statistical test is of 0.1 or lower, preferably 0.05, 0.01, 0.001 or lower.

By "decrease" or "reduction" is referred herein as a reduction in at least a 10%, 20%, 30%, 40%, 50%, 60%, 70%, 80%, 90%, or 100% in comparison to a reference cell or value or reference tissue. By "reference cell or tissue" is referred herein to a cell or tissue not treated with the sEVs of exosomes described herein. Preferably, said cell or tissue is a mammalian cell e.g., a human cell, preferably a neuron or a cell or tissue from the nervous system. The reference cell may also be a single cell or a population of cells derived from the same subject or from a population of subjects. By "reference value" is referred herein to the average known and correct measurement of a parameter, for instance activity levels of AMPKα2 protein in untreated cells or in reference cells. Reference values are an average of repeated measurements from more accurate measuring equipment. A skilled artisan would know how to obtain said reference value.

The levels of AMPKα2 enzymatic activity may be expressed or measured by an "absolute" quantification or by a "relative" or comparative quantification and they can be calculated by a skilled person using suitable techniques known in the art. For example western blot, Immunochestry, Immunofluorescence, qPCR, etc.

The population may be part of a composition comprising emulsions containing sEVs or exosomes. Such emulsions may comprise oil-in-water emulsions. Thus, the first aspect of the invention also includes compositions comprising the sEVs or exosomes of the invention, preferably together with one or more pharmaceutically acceptable carriers.

The compositions, preferably emulsions, may be made by means known in the art. They may be made from freshly prepared sEVs or exosomes, or lyophilised sEVs or exosomes or sEVs or exosomes stored in oil. Emulsions are heterogeneous systems composed of at least two immiscible liquids, for example water and oil, one of which is usually uniformly dispersed as fine droplets throughout the other liquid phase by a mechanical agitation process. The composition may further comprise at least one emulsifier. An emulsifier (also known as an "emulgent") is a substance that stabilizes an emulsion by increasing its kinetic stability.

The carriers are biologically acceptable without eliciting an adverse reaction (e.g. immune response) when administered to the host. Suitable pharmaceutically acceptable carriers are well known in the art and vary with the desired form and mode of administration of the pharmaceutical formulation. For example, they may include diluents or excipients such as fillers, binders, wetting agents, disintegrators, surface-active agents, lubricants and the like. Typically, the carrier is a solid, a liquid or a vaporizable carrier, or a combination thereof. Each carrier should be "acceptable" in the sense of being compatible with the other ingredients in the formulation and not injurious to the subject.

The sEVs or exosomes may be conveniently stored as a suspension or dispersion in oil. For this purpose, freshly prepared sEVs or exosomes or lyophilised exosomes may be suspended or dispersed in oil. The oil may comprise any suitable oil, such as a plant oil. The sEVs or exosomes may be suspended or dispersed in for example olive oil, palm oil, soy oil or coconut oil. The sEVs or exosomes may be suspended or dispersed in the oil in any suitable proportion.

The sEVs or exosomes suspension or dispersion in oil may be stored, for example at room temperature, prior to use. The sEVs or exosomes suspension or dispersion in oil may be such that the exosome exhibits at least one biological activity of an sEVs or exosomes following a period of storage or after a period of storage. The biological activity may comprise a therapeutic activity, such as correcting, reducing, ameliorating, treating, preventing ischemia, as will be described below. The biological activity may be derived from the presence of a dominant negative AMPKα2 protein (AMPKα2-DN), which in turn decreases AMPK activity and pACC phosphorylation. The biological activity may be derived from the reduced levels of phosphorylation of acetyl-CoA carboxylase alpha (pACCα).

Method for producing the population or the composition or pharmaceutical composition according to the present invention are known in the art, and they optionally comprise the step of specifically enriching for substantially pure populations of sEVs or exosomes. For this, generally any suitable method for purifying and/or enriching can be used, such as methods comprising magnetic particles, filtration, dialysis, ultracentrifugation, ExoQuick^{™} (Systems Biosciences, CA, USA), and/or chromatography.

In an embodiment, the composition is a pharmaceutical composition. The pharmaceutical composition is preferably enriched with the population of sEVs exosomes having a size of between about 30 to 200 nm, preferably 30 to 150 nm in size. The sEVs or exosomes amount can be measured by protein amount, for example, by using a Bradford assay (BioRad) or a BCA protein assay kit (Pierce). Yet, the optimal dose will be selected according to the administration route, treatment regime and/or administration schedule, having regard to the existing toxicity and effectiveness data.

In **a second aspect**, the present invention provides the use of the population according to the first aspect or any of its embodiments, including the composition or pharmaceutical composition, in therapy or for the generation of a medicament.

Preferably, the use is a therapeutic and/or prophylactic treatment of ischemia or ischemia-reperfusion in a subject in need thereof. The term "treatment" as used herein refers to the medical care given to a patient for a disease or injury. This term includes curing the disease but also ameliorating, mitigating, reducing or preventing the symptoms of said disease.

In an embodiment, the population or composition according to the first aspect or any of its embodiments is used in the therapeutic treatment of ischemia or ischemia-reperfusion in a subject in need thereof. The term "therapeutic treatment" as used herein refers to bringing a body from a pathological state or disease back to its normal, healthy state. The term "therapeutic treatment" also refers to the mitigation, amelioration, or reduction of the harmful effect of ischemia in a subject, or in some manner reducing the symptoms associated with such effects.

In an embodiment, the population or composition according to the first aspect or any of its embodiments is used in the prophylactic treatment of ischemia or ischemia-reperfusion in a subject in need thereof. The term "prophylactic treatment" as used herein refers to preventing a pathological stage derived from ischemia.

In an embodiment, the population or composition according to the first aspect or any of its embodiments is used in correcting, reducing, ameliorating, treating, and/or preventing ischemia. The term "ischemia" refers to a pathological stage in which there is a period of lack of oxygen in the target cell or tissue. The period of lack of oxygen may be a few seconds or even minutes. The period of lack of oxygen may be characterized by being a period of hypoxia or anoxia. As used herein, the term "hypoxia" refers to the presence of less than normal amounts of O₂ than those present in a vertebrate or in its blood, cells or tissue. As used herein, the term "anoxia" refers to the lack of O₂.

The ischemia may be caused by an inadequate blood flow. The ischemia may be from any cause including acute coronary syndromes (such as myocardial infarction, coronary revascularization, coronary bypass surgery and coronary angioplastyistent placement); stroke, renal, retinal, mesenteric or limb ischemia due to vascular occlusion; organ transplant surgery (for maintaining viability and function of the transplanted organ); ischemia associated with vascular surgery (including hypothermic arrest and vascular cross-clamping); ischemic reperfusion injury (such as coronary or cerebral reperfusion injury); cerebrovascular accidents (such as a thromboembolic or hemorrhagic stroke) that can lead to ischemia in the brain; operative ischemia; traumatic hemorrhage (for example, a hypovolemic stroke that can lead to CNS hypoxia or anoxia), or resuscitation injury. Preferably, the ischemia is caused by myocardial infarction or is cerebrovascular ischemia. Preferably, the ischemia treated or prevented, preferably treated, is ischemic stroke.

The used described herein preferably comprises administering an effective amount of a substantially pure population of the sEVs or exosomes of the invention, together with one or more pharmaceutically acceptable carriers. An "effective amount" is the amount sufficient to reduce, ameliorate, treat and/or prevent ischemia. The effective amount will vary depending upon several factors including the age and weight of the subject to be treated, how advanced the disease state is, the general health of the patient, the severity of the symptoms.

As used herein, the phrase "subject in need thereof" includes subjects, such as mammalian subjects, preferably humans, that would benefit from administration of the population of sEVs or exosomes, or the compositions comprising them, disclosed herein. The subject may be a female, preferably human female. Preferably, the subject is a male, more preferably a human male. More preferably, the subject in need of a treatment for ischemia. The terms "individual", "patient" or "subject" are used interchangeably in the present application and are not meant to be limiting in any way. The "individual", "patient" or "subject" can be of any age, sex and physical condition.

The administration route can be systemic or local. By "systemic administration route" is referred herein the administration of the composition into the circulatory system, directly or indirectly. Systemic administration routes comprise parenteral route such as intravascular, intravenous, intraarterial, subcutaneous, intramuscular, intraperitoneal, intraventricular, intraepidural, or others as well as oral, nasal, ophthalmic, or rectal. A preferred systemic route of administration is intravascular, which is herein understood as the administration within a vessel or vessels and typically includes intravenous or intraarterial administration.

The use provided herein may be short or long term defined as a period of time exceeding three months.

In a preferred embodiment, the use of the population of sEVs of the invention, preferably exosomes of the invention, is in combination with nicotine, wherein the combination is herein understood as the simultaneous or sequential administration, in any order, of a) the sEVs of the invention, and b) nicotine. Preferably, the use in combination comprises the simultaneous or sequential administration, in any order, of a) the exosomes of the invention, and b) nicotine. Preferably, the use in combination comprises the simultaneous or sequential administration, in any order, of a) the exosomes of the invention comprising a gene encoding for a protein as set forth in SEQ ID NO: 2, and b) nicotine.

In a **third aspect,** the present invention provides a kit comprising the sEvs or the exosomes as defined in the first aspect or any of its embodiments, or a composition, preferably a pharmaceutical composition, comprising them. Preferably, the kit further comprises nicotine for the combined administration as defined above.

### SEQUENCE LISTING

**SEQ ID NO: 1:** wildtype APMKa2 
MAEKQHDGRVKIGHYVLGDTLGVGTFGKVKIGEHQLTGHKVAVKILNRQKIRSLDVVGKIKREIQNLKL
FRHPHIIKLYQVISTPTDFFMVMEYVSGGELFDYICKHGRVEEVEARRLFQQILSAVDYCHRHMVVHRDL
KPENVLLDAQMNAKIADFGLSNMMSDGEFLRTSCGSPNYAAPEVISGRLYAGPEVDIWSCGVILYALLCG
TLPFDDEHVPTLFKKIRGGVFYIPDYLNRSVATLLMHMLQVDPLKRATIKDIREHEWFKQDLPSYLFPED
PSYDANVIDDEAVKEVCEKFECTESEVMNSLYSGDPQDQLAVAYHLIIDNRRIMNQASEFYLASSPPSGS
FMDDSAMHIPPGLKPHPERMPPLIADSPKARCPLDALNTTKPKSLAVKKAKWHLGIRSQSKACDIMAEV
YRAMKQLGFEWKVVNAYHLRVRRKNPVTGNYVKMSLQLYLVDSRSYLLDFKSIDDEVVEQRSGSSTPQR
SCSAAGLHRARSSFDSSTAENHSLSGSLTGSLTGSTLSSASPRLGSHTMDFFEMCASLITALAR
**SEQ ID NO: 2:** AMPKα2-DN mutant protein amino acid sequence:
   Underlined : Myc tag
   bold: Linker
   bold and underlined: D->A substitution 
MEQKLISEEDLGGGEKQKHDGRVKIGHYVLGDTLGVGTFGKVKIGEHQLTGHKVAVKILNRQKIRSLDVV
GKIKREIQNLKLFRHPHIIKLYQVISTPTDFFMVMEYVSGGELFDYICKHGRVEEVEARRLFQQILSAVDYCH
RHMVVHRDLKPENVLLDAQMNAKIAAFGLSNMMSDGEFLRTSCGSPNYAPEVISGRLYAGPEVDIWSCG
VILYALLCGTLPFDDEHVPTLFKKIRGGVFYIPEYLNRSIATLLMHMLQVDPLKRATIKDIREHEWFKQDLPSYL
FPEDPSYDANVIDDEAVKEVCEKFECTESEVMNSLYSGDPQDQLAVAYHLIIDNRRIMNQASEFYLASSPPTGS
FMDDSAMHIPPGLKPHPERMPPLIADSPKARCPLDALNTTKPKSLAVKKAKWHLGIRSQSKPYDIMAEVYRA
MKQLDFEWKVVNAYHLRVRRKNPVTGNYVKMSLQLYLVDNRSYLLDFKSIDDEVVEQRSGSSTPQRSCSAAG
LHRPRSSVDSSTAENHSLSGSLTGSLTGSTLSSASPRLGSHTMDFFEMCASLITALAR
**SEQ ID NO: 3:** AMPKa2-DN mutant nucleotide sequence: 
GGCGAGAAGC AGAAGCACGA CGGGCGTGTG AAGATCGGAC ACTACGTGCT
GGGGGACACC CTGGGCGTCG GCACCTTCGG CAAAGTGAAG ATTGGAGAAC
ATCAATTGAC AGGCCATAAA GTGGCAGTTA AGATCTTAAA TAGACAGAAG
ATTCGCAGTT TAGATGTTGT TGGAAAAATA AAACGAGAAA TTCAAAATCT
TAAACTCTTT CGTCATCCTC ATATTATCAA ACTCTACCAA GTGATCAGCA
CTCCAACAGA CTTTTTTATG GTAATGGAAT ATGTGTCTGG AGGTGAATTG
TTCGACTACA TCTGTAAACA CGGGAGGGTT GAAGAGGTGG AAGCTCGCCG
GCTCTTCCAG CAGATTCTGT CTGCCGTGGA CTACTGTCAC AGGCACATGG
TTGTCCACAG GGACCTGAAG CCAGAGAACG TGTTGCTGGA CGCCCAGATG
AATGCTAAGA TAGCTGCCTT CGGACTCTCT AATATGATGT CAGATGGTGA
ATTTCTACGA ACTAGCTGTG GATCGCCAAA TTATGCAGCA CCGGAGGTCA
TCTCAGGAAG GCTGTATGCG GGTCCTGAGG TTGATATCTG GAGCTGTGGT
GTTATCCTGT ATGCCCTTCT CTGTGGCACC CTCCCGTTCG ACGATGAGCA
 
CGTGCCTACG CTCTTTAAGA AGATCCGAGG GGGTGTGTTC TACATCCCGG
AGTATCTCAA CCGTTCTATT GCCACTCTGC TGATGCACAT GCTGCAGGTG
GACCCCTTGA AGCGAGCAAC TATCAAAGAC ATACGAGAGC ATGAATGGTT
CAAGCAAGAT TTGCCCAGTT ACCTCTTTCC TGAAGACCCC TCCTATGATG
CTAACGTCAT TGATGATGAG GCTGTGAAAG AAGTATGTGA AAAATTTGAG
TGTACAGAAT CAGAAGTGAT GAACAGTTTA TACAGTGGTG ACCCTCAAGA
CCAGCTCGCA GTGGCTTATC ATCTCATCAT TGACAATCGG AGAATAATGA
ACCAAGCCAG TGAGTTCTAC CTCGCCTCCA GTCCTCCAAC GGGTTCCTTC
ATGGACGATA GTGCCATGCA TATTCCCCCC GGCCTGAAAC CACATCCTGA
AAGGATGCCA CCTCTCATAG CAGACAGCCC CAAAGCACGC TGTCCACTGG
ATGCACTCAA CACAACTAAG CCCAAATCTT TAGCTGTGAA AAAAGCCAAG
TGGCACCTTG GGATCCGAAG CCAGAGCAAA CCATACGACA TTATGGCGGA
GGTGTACCGA GCTATGAAGC AGCTGGACTT TGAATGGAAG GTAGTGAATG
CATACCATCT TCGAGTAAGA AGAAAAAACC CAGTGACTGG CAATTACGTG
AAAATGAGCT TACAGCTTTA CCTGGTTGAC AATCGGAGCT ATCTTCTAGA
CTTTAAAAGC ATCGATGATG AGGTGGTGGA GCAGAGGTCT GGTTCTTCAA
CACCTCAGCG CTCCTGTTCT GCTGCCGGCC TCCACAGACC TCGGTCAAGT
GTCGATTCCA GCACAGCCGA GAACCATTCA CTGTCTGGCT CTCTCACTGG
TTCTTTGACT GGCAGCACTT TGTCCTCCGC TTCCCCGCGC CTGGGCAGTC
ATACCATGGA TTTTTTTGAA ATGTGCGCCA GTCTTATCAC TGCTTTAGCC
CGTTGATAAT AACTCAC
**SEQ ID NO: 4:** CMV promoter nucleotide sequence: 
TACGGTAAAT GGCCCGCCTG GCTGACCGCC CAACGACCCC CGCCCATTGA
CGTCAATAAT GACGTATGTT CCCATAGTAA CGCCAATAGG GACTTTCCAT
TGACGTCAAT GGGTGGAGTA TTTACGGTAA ACTGCCCACT TGGCAGTACA
TCAAGTGTAT CATATGCCAA GTACGCCCCC TATTGACGTC AATGACGGTA
AATGGCCCGC CTGGCATTAT GCCCAGTACA TGACCTTATG GGACTTTCCT
ACTTGGCAGT ACATCTACGT ATTAGTCATC GCTATTACCA TGGTGATGCG
GTTTTGGCAG TACATCAATG GGCGTGGATA GCGGTTTGAC TCACGGGGAT
TTCCAAGTCT CCACCCCATT GACGTCAATG GGAGTTTGTT TTGGCACCAA
AATCAACGGG ACTTTCCAAA ATGTCGTAAC AACTCCGCCC CATTGACGCA
AATGGGCGGT AGGCGTGTAC GGTGGGAGGT CTATATAAGC AGAGCTCGTT
**SEQ ID NO: 5:** neurotrophic rabies virus (RVG) peptide fused to lysosome-associated membrane protein 2b (Lamp2b). Note:
   (Not underlined: Lamp2b sequence)
   (Underlined: RVG sequence) 
MCLSPVKGAKLILIFLFLGAVQSNALIVNLTDSKGTCLYARYTIWMPENPRPGTPCDIFTNSR
GKRASNGSGGAEWEMNFTITYETTNQTNKTITIAVPDKATHDGSSCGDDRNSAKIMIQFGFA
VSWAVNFTKEASHYSIHDIVLSYNTSDSTVFPGAVAKGVHTVKNPENFKVPLDVIFKCNSVLT
YNLTPVVQKYWGIHLQAFVQNGTVSKNEQVCEEDQTPTTVAPIIHTTAPSTTTTLTPTSTPTP
TPTPTPTVGNYSIRNGNTTCLLATMGLQLNITEEKVPFIFNINPATTNFTGSCQPQSAQLRLN
NSQIKYLDFIFAVKNEKRFYLKEVNVYMYLANGSAFNISNKNLSFWDAPLGSSYMCNKEQVL
 
SVSRAFQINTFNLKVQPFNVTKGQYSTAQECSLDDDTILIPIIVGAGLSGLIIVIVIAYLIGRRKT
YAGYQTL
**SEQ ID NO: 6:** VQAd CMV AMPKa2DN 31487, see Fig. 10 
   1 TTAAGCTAGC ATCATCAATA ATATACCTTA TTTTGGATTG AAGCCAATAT
  51 GATAATGAGG GGGTGGAGTT TGTGACGTGG CGCGGGGCGT GGGAACGGGG
 101 CGGGTGACGT AGTAGTGTGG CGGAAGTGTG ATGTTGCAAG TGTGGCGGAA
 151 CACATGTAAG CGACGGATGT GGCAAAAGTG ACGTTTTTGG TGTGCGCCGG
 201 TGTACACAGG AAGTGACAAT TTTCGCGCGG TTTTAGGCGG ATGTTGTAGT
 251 AAATTTGGGC GTAACCGAGT AAGATTTGGC CATTTTCGCG GGAAAACTGA
 301 ATAAGAGGAA GTGAAATCTG AATAATTTTG TGTTACTCAT AGCGCGTAAT
 351 ATTTGTCTAG GGAGATCAGC CTGCAGGTCG TTACATAACT TACGGTAAAT
 401 GGCCCGCCTG GCTGACCGCC CAACGACCCC CGCCCATTGA CGTCAATAAT
 451 GACGTATGTT CCCATAGTAA CGCCAATAGG GACTTTCCAT TGACGTCAAT
 501 GGGTGGAGTA TTTACGGTAA ACTGCCCACT TGGCAGTACA TCAAGTGTAT
 551 CATATGCCAA GTACGCCCCC TATTGACGTC AATGACGGTA AATGGCCCGC
 601 CTGGCATTAT GCCCAGTACA TGACCTTATG GGACTTTCCT ACTTGGCAGT
 651 ACATCTACGT ATTAGTCATC GCTATTACCA TGGTGATGCG GTTTTGGCAG
 701 TACATCAATG GGCGTGGATA GCGGTTTGAC TCACGGGGAT TTCCAAGTCT
 751 CCACCCCATT GACGTCAATG GGAGTTTGTT TTGGCACCAA AATCAACGGG
 801 ACTTTCCAAA ATGTCGTAAC AACTCCGCCC CATTGACGCA AATGGGCGGT
 851 AGGCGTGTAC GGTGGGAGGT CTATATAAGC AGAGCTCGTT TAGTGAACCG
 901 TCAGATGGTA CCGTTTAAAC TCGAGGTCGA CGGTATCGAT AAGCTTGATA
 951 TCGAATTCGC CATGGAGCAG AAGCTTATCT CCGAGGAGGA CCTCGGTGGC
1001 GGCGAGAAGC AGAAGCACGA CGGGCGTGTG AAGATCGGAC ACTACGTGCT
1051 GGGGGACACC CTGGGCGTCG GCACCTTCGG CAAAGTGAAG ATTGGAGAAC
1101 ATCAATTGAC AGGCCATAAA GTGGCAGTTA AGATCTTAAA TAGACAGAAG
1151 ATTCGCAGTT TAGATGTTGT TGGAAAAATA AAACGAGAAA TTCAAAATCT
1201 TAAACTCTTT CGTCATCCTC ATATTATCAA ACTCTACCAA GTGATCAGCA
1251 CTCCAACAGA CTTTTTTATG GTAATGGAAT ATGTGTCTGG AGGTGAATTG
1301 TTCGACTACA TCTGTAAACA CGGGAGGGTT GAAGAGGTGG AAGCTCGCCG
1351 GCTCTTCCAG CAGATTCTGT CTGCCGTGGA CTACTGTCAC AGGCACATGG
1401 TTGTCCACAG GGACCTGAAG CCAGAGAACG TGTTGCTGGA CGCCCAGATG
1451 AATGCTAAGA TAGCTGCCTT CGGACTCTCT AATATGATGT CAGATGGTGA
1501 ATTTCTACGA ACTAGCTGTG GATCGCCAAA TTATGCAGCA CCGGAGGTCA
1551 TCTCAGGAAG GCTGTATGCG GGTCCTGAGG TTGATATCTG GAGCTGTGGT
1601 GTTATCCTGT ATGCCCTTCT CTGTGGCACC CTCCCGTTCG ACGATGAGCA
1651 CGTGCCTACG CTCTTTAAGA AGATCCGAGG GGGTGTGTTC TACATCCCGG
1701 AGTATCTCAA CCGTTCTATT GCCACTCTGC TGATGCACAT GCTGCAGGTG
1751 GACCCCTTGA AGCGAGCAAC TATCAAAGAC ATACGAGAGC ATGAATGGTT
1801 CAAGCAAGAT TTGCCCAGTT ACCTCTTTCC TGAAGACCCC TCCTATGATG
1851 CTAACGTCAT TGATGATGAG GCTGTGAAAG AAGTATGTGA AAAATTTGAG
1901 TGTACAGAAT CAGAAGTGAT GAACAGTTTA TACAGTGGTG ACCCTCAAGA
1951 CCAGCTCGCA GTGGCTTATC ATCTCATCAT TGACAATCGG AGAATAATGA
2001 ACCAAGCCAG TGAGTTCTAC CTCGCCTCCA GTCCTCCAAC GGGTTCCTTC
2051 ATGGACGATA GTGCCATGCA TATTCCCCCC GGCCTGAAAC CACATCCTGA
2101 AAGGATGCCA CCTCTCATAG CAGACAGCCC CAAAGCACGC TGTCCACTGG
2151 ATGCACTCAA CACAACTAAG CCCAAATCTT TAGCTGTGAA AAAAGCCAAG
2201 TGGCACCTTG GGATCCGAAG CCAGAGCAAA CCATACGACA TTATGGCGGA
2251 GGTGTACCGA GCTATGAAGC AGCTGGACTT TGAATGGAAG GTAGTGAATG
2301 CATACCATCT TCGAGTAAGA AGAAAAAACC CAGTGACTGG CAATTACGTG
2351 AAAATGAGCT TACAGCTTTA CCTGGTTGAC AATCGGAGCT ATCTTCTAGA
2401 CTTTAAAAGC ATCGATGATG AGGTGGTGGA GCAGAGGTCT GGTTCTTCAA
 
2451 CACCTCAGCG CTCCTGTTCT GCTGCCGGCC TCCACAGACC TCGGTCAAGT
2501 GTCGATTCCA GCACAGCCGA GAACCATTCA CTGTCTGGCT CTCTCACTGG
2551 TTCTTTGACT GGCAGCACTT TGTCCTCCGC TTCCCCGCGC CTGGGCAGTC
2601 ATACCATGGA TTTTTTTGAA ATGTGCGCCA GTCTTATCAC TGCTTTAGCC
2651 CGTTGATAAT AACTCACCAC CGGCTAGAGC GGCCGCCACA GCGGGGAGAT
2701 CCAGACATGA TAAGATACAT TGATGAGTTT GGACAAACCA CAACTAGAAT
2751 GCAGTGAAAA AAATGCTTTA TTTGTGAAAT TTGTGATGCT ATTGCTTTAT
2801 TTGTAACCAT TATAAGCTGC AATAAACAAG TTAACAACAA CAATTGCATT
2851 CATTTTATGT TTCAGGTTCA GGGGGAGGTG TGGGAGGTTT TTTAAAGCAA
2901 GTAAAACCTC TACAAATGTG GTATGGCTGA TTATGATCCG GCTGCCTCGC
2951 GCGTTTCGGT GATGACGGTG AAAACCTCTG ACACATGCAG CTCCCGGAGA
3001 CGGTCACAGC TTGTCTGTAA GCGGATGCCG GGAGCAGACA AGCCCGTCAG
3051 GGCGCGTCAG CGGGTGTTGG CGGGTGTCGG GGCGCAGCCA TGAGGTCGAC
3101 TCTAGTCCCC GCGGTGGCAG ATCTGGAAGG TGCTGAGGTA CGATGAGACC
3151 CGCACCAGGT GCAGACCCTG CGAGTGTGGC GGTAAACATA TTAGGAACCA
3201 GCCTGTGATG CTGGATGTGA CCGAGGAGCT GAGGCCCGAT CACTTGGTGC
3251 TGGCCTGCAC CCGCGCTGAG TTTGGCTCTA GCGATGAAGA TACAGATTGA
3301 GGTACTGAAA TGTGTGGGCG TGGCTTAAGG GTGGGAAAGA ATATATAAGG
3351 TGGGGGTCTT ATGTAGTTTT GTATCTGTTT TGCAGCAGCC GCCGCCGCCA
3401 TGAGCACCAA CTCGTTTGAT GGAAGCATTG TGAGCTCATA TTTGACAACG
3451 CGCATGCCCC CATGGGCCGG GGTGCGTCAG AATGTGATGG GCTCCAGCAT
3501 TGATGGTCGC CCCGTCCTGC CCGCAAACTC TACTACCTTG ACCTACGAGA
3551 CCGTGTCTGG AACGCCGTTG GAGACTGCAG CCTCCGCCGC CGCTTCAGCC
3601 GCTGCAGCCA CCGCCCGCGG GATTGTGACT GACTTTGCTT TCCTGAGCCC
3651 GCTTGCAAGC AGTGCAGCTT CCCGTTCATC CGCCCGCGAT GACAAGTTGA
3701 CGGCTCTTTT GGCACAATTG GATTCTTTGA CCCGGGAACT TAATGTCGTT
3751 TCTCAGCAGC TGTTGGATCT GCGCCAGCAG GTTTCTGCCC TGAAGGCTTC
3801 CTCCCCTCCC AATGCGGTTT AAAACATAAA TAAAAAACCA GACTCTGTTT
3851 GGATTTGGAT CAAGCAAGTG TCTTGCTGTC TTTATTTAGG GGTTTTGCGC
3901 GCGCGGTAGG CCCGGGACCA GCGGTCTCGG TCGTTGAGGG TCCTGTGTAT
3951 TTTTTCCAGG ACGTGGTAAA GGTGACTCTG GATGTTCAGA TACATGGGCA
4001 TAAGCCCGTC TCTGGGGTGG AGGTAGCACC ACTGCAGAGC TTCATGCTGC
4051 GGGGTGGTGT TGTAGATGAT CCAGTCGTAG CAGGAGCGCT GGGCGTGGTG
4101 CCTAAAAATG TCTTTCAGTA GCAAGCTGAT TGCCAGGGGC AGGCCCTTGG
4151 TGTAAGTGTT TACAAAGCGG TTAAGCTGGG ATGGGTGCAT ACGTGGGGAT
4201 ATGAGATGCA TCTTGGACTG TATTTTTAGG TTGGCTATGT TCCCAGCCAT
4251 ATCCCTCCGG GGATTCATGT TGTGCAGAAC CACCAGCACA GTGTATCCGG
4301 TGCACTTGGG AAATTTGTCA TGTAGCTTAG AAGGAAATGC GTGGAAGAAC
4351 TTGGAGACGC CCTTGTGACC TCCAAGATTT TCCATGCATT CGTCCATAAT
4401 GATGGCAATG GGCCCACGGG CGGCGGCCTG GGCGAAGATA TTTCTGGGAT
4451 CACTAACGTC ATAGTTGTGT TCCAGGATGA GATCGTCATA GGCCATTTTT
4501 ACAAAGCGCG GGCGGAGGGT GCCAGACTGC GGTATAATGG TTCCATCCGG
4551 CCCAGGGGCG TAGTTACCCT CACAGATTTG CATTTCCCAC GCTTTGAGTT
4601 CAGATGGGGG GATCATGTCT ACCTGCGGGG CGATGAAGAA AACGGTTTCC
4651 GGGGTAGGGG AGATCAGCTG GGAAGAAAGC AGGTTCCTGA GCAGCTGCGA
4701 CTTACCGCAG CCGGTGGGCC CGTAAATCAC ACCTATTACC GGGTGCAACT
4751 GGTAGTTAAG AGAGCTGCAG CTGCCGTCAT CCCTGAGCAG GGGGGCCACT
4801 TCGTTAAGCA TGTCCCTGAC TCGCATGTTT TCCCTGACCA AATCCGCCAG
4851 AAGGCGCTCG CCGCCCAGCG ATAGCAGTTC TTGCAAGGAA GCAAAGTTTT
4901 TCAACGGTTT GAGACCGTCC GCCGTAGGCA TGCTTTTGAG CGTTTGACCA
4951 AGCAGTTCCA GGCGGTCCCA CAGCTCGGTC ACCTGCTCTA CGGCATCTCG
5001 ATCCAGCATA TCTCCTCGTT TCGCGGGTTG GGGCGGCTTT CGCTGTACGG
5051 CAGTAGTCGG TGCTCGTCCA GACGGGCCAG GGTCATGTCT TTCCACGGGC
5101 GCAGGGTCCT CGTCAGCGTA GTCTGGGTCA CGGTGAAGGG GTGCGCTCCG
 
5151 GGCTGCGCGC TGGCCAGGGT GCGCTTGAGG CTGGTCCTGC TGGTGCTGAA
5201 GCGCTGCCGG TCTTCGCCCT GCGCGTCGGC CAGGTAGCAT TTGACCATGG
5251 TGTCATAGTC CAGCCCCTCC GCGGCGTGGC CCTTGGCGCG CAGCTTGCCC
5301 TTGGAGGAGG CGCCGCACGA GGGGCAGTGC AGACTTTTGA GGGCGTAGAG
5351 CTTGGGCGCG AGAAATACCG ATTCCGGGGA GTAGGCATCC GCGCCGCAGG
5401 CCCCGCAGAC GGTCTCGCAT TCCACGAGCC AGGTGAGCTC TGGCCGTTCG
5451 GGGTCAAAAA CCAGGTTTCC CCCATGCTTT TTGATGCGTT TCTTACCTCT
5501 GGTTTCCATG AGCCGGTGTC CACGCTCGGT GACGAAAAGG CTGTCCGTGT
5551 CCCCGTATAC AGACTTGAGA GGCCTGTCCT CGACCGATGC CCTTGAGAGC
5601 CTTCAACCCA GTCAGCTCCT TCCGGTGGGC GCGGGGCATG ACTATCGTCG
5651 CCGCACTTAT GACTGTCTTC TTTATCATGC AACTCGTAGG ACAGGTGCCG
5701 GCAGCGCTCT GGGTCATTTT CGGCGAGGAC CGCTTTCGCT GGAGCGCGAC
5751 GATGATCGGC CTGTCGCTTG CGGTATTCGG AATCTTGCAC GCCCTCGCTC
5801 AAGCCTTCGT CACTGGTCCC GCCACCAAAC GTTTCGGCGA GAAGCAGGCC
5851 ATTATCGCCG GCATGGCGGC CGACGCGCTG GGCTACGTCT TGCTGGCGTT
5901 CGCGACGCGA GGCTGGATGG CCTTCCCCAT TATGATTCTT CTCGCTTCCG
5951 GCGGCATCGG GATGCCCGCG TTGCAGGCCA TGCTGTCCAG GCAGGTAGAT
6001 GACGACCATC AGGGACAGCT TCAAGGCCAG CAAAAGGCCA GGAACCGTAA
6051 AAAGGCCGCG TTGCTGGCGT TTTTCCATAG GCTCCGCCCC CCTGACGAGC
6101 ATCACAAAAA TCGACGCTCA AGTCAGAGGT GGCGAAACCC GACAGGACTA
6151 TAAAGATACC AGGCGTTTCC CCCTGGAAGC TCCCTCGTGC GCTCTCCTGT
6201 TCCGACCCTG CCGCTTACCG GATACCTGTC CGCCTTTCTC CCTTCGGGAA
6251 GCGTGGCGCT TTCTCATAGC TCACGCTGTA GGTATCTCAG TTCGGTGTAG
6301 GTCGTTCGCT CCAAGCTGGG CTGTGTGCAC GAACCCCCCG TTCAGCCCGA
6351 CCGCTGCGCC TTATCCGGTA ACTATCGTCT TGAGTCCAAC CCGGTAAGAC
6401 ACGACTTATC GCCACTGGCA GCAGCCACTG GTAACAGGAT TAGCAGAGCG
6451 AGGTATGTAG GCGGTGCTAC AGAGTTCTTG AAGTGGTGGC CTAACTACGG
6501 CTACACTAGA AGGACAGTAT TTGGTATCTG CGCTCTGCTG AAGCCAGTTA
6551 CCTTCGGAAA AAGAGTTGGT AGCTCTTGAT CCGGCAAACA AACCACCGCT
6601 GGTAGCGGTG GTTTTTTTGT TTGCAAGCAG CAGATTACGC GCAGAAAAAA
6651 AGGATCTCAA GAAGATCCTT TGATCTTTTC TACGGGGTCT GACGCTCAGT
6701 GGAACGAAAA CTCACGTTAA GGGATTTTGG TCATGAGATT ATCAAAAAGG
6751 ATCTTCACCT AGATCCTTTT AAATTAAAAA TGAAGTTTTA AATCAATCTA
6801 AAGTATATAT GAGTAAACTT GGTCTGACAG TTACCAATGC TTAATCAGTG
6851 AGGCACCTAT CTCAGCGATC TGTCTATTTC GTTCATCCAT AGTTGCCTGA
6901 CTCCCCGTCG TGTAGATAAC TACGATACGG GAGGGCTTAC CATCTGGCCC
6951 CAGTGCTGCA ATGATACCGC GAGACCCACG CTCACCGGCT CCAGATTTAT
7001 CAGCAATAAA CCAGCCAGCC GGAAGGGCCG AGCGCAGAAG TGGTCCTGCA
7051 ACTTTATCCG CCTCCATCCA GTCTATTAAT TGTTGCCGGG AAGCTAGAGT
7101 AAGTAGTTCG CCAGTTAATA GTTTGCGCAA CGTTGTTGCC ATTGCTGCAG
7151 GCATCGTGGT GTCACGCTCG TCGTTTGGTA TGGCTTCATT CAGCTCCGGT
7201 TCCCAACGAT CAAGGCGAGT TACATGATCC CCCATGTTGT GCAAAAAAGC
7251 GGTTAGCTCC TTCGGTCCTC CGATCGTTGT CAGAAGTAAG TTGGCCGCAG
7301 TGTTATCACT CATGGTTATG GCAGCACTGC ATAATTCTCT TACTGTCATG
7351 CCATCCGTAA GATGCTTTTC TGTGACTGGT GAGTACTCAA CCAAGTCATT
7401 CTGAGAATAG TGTATGCGGC GACCGAGTTG CTCTTGCCCG GCGTCAACAC
7451 GGGATAATAC CGCGCCACAT AGCAGAACTT TAAAAGTGCT CATCATTGGA
7501 AAACGTTCTT CGGGGCGAAA ACTCTCAAGG ATCTTACCGC TGTTGAGATC
7551 CAGTTCGATG TAACCCACTC GTGCACCCAA CTGATCTTCA GCATCTTTTA
7601 CTTTCACCAG CGTTTCTGGG TGAGCAAAAA CAGGAAGGCA AAATGCCGCA
7651 AAAAAGGGAA TAAGGGCGAC ACGGAAATGT TGAATACTCA TACTCTTCCT
7701 TTTTCAATAT TATTGAAGCA TTTATCAGGG TTATTGTCTC ATGAGCGGAT
7751 ACATATTTGA ATGTATTTAG AAAAATAAAC AAATAGGGGT TCCGCGCACA
7801 TTTCCCCGAA AAGTGCCACC TGACGTCTAA GAAACCATTA TTATCATGAC
 
7851 ATTAACCTAT AAAAATAGGC GTATCACGAG GCCCTTTCGT CTTCAAGAAA
7901 ATTAA
**SEQ ID NO: 7**: HIF nucleotide sequence
   ACGTGNNNNACGTG
**SEQ ID NO: 8**: AMP-activated, alpha 2 catalytic subunit (Prkaa2), transcript variant 1, mRNA *Mus musculus* 
GGGTAGGCGGCGGCGGCGGTGGCGGCGGTAGCGGCGGCAGCGGTAGCGGCGGCGCTCAGAG
CCCGCGGCAGGGGGAGCCCGTGCGCCGAACATGGCTGAGAAGCAGAAGCACGACGGGCGGGTGAAGATCG
GACACTACGTCCTGGGGGACACCCTGGGCGTCGGCACCTTCGGCAAAGTGAAGATTGGAGAACACCAATT
GACAGGCCATAAAGTGGCAGTTAAGATCTTAAATAGACAGAAGATTCGCAGTTTAGATGTTGTTGGAAAA
ATAAAACGAGAAATTCAAAATCTTAAACTCTTTCGTCATCCTCATATTATCAAACTCTACCAGGTGATCA
GCACTCCGACAGACTTTTTTATGGTAATGGAATATGTGTCTGGAGGTGAATTGTTCGACTACATCTGCAA
ACATGGGCGGGTTGAAGAGGTGGAAGCGCGCCGGCTCTTCCAGCAGATCCTGTCTGCCGTGGATTACTGT
CACAGGCATATGGTTGTCCATAGGGACCTGAAGCCAGAGAATGTGCTGCTGGATGCCCAGATGAACGCTA
AGATAGCTGACTTTGGACTATCTAATATGATGTCAGATGGTGAATTTCTACGAACTAGCTGTGGATCGCC
AAATTATGCAGCACCTGAGGTCATCTCAGGAAGGCTGTATGCAGGTCCCGAGGTCGATATCTGGAGCTGT
GGTGTCATCCTGTATGCCCTTCTCTGTGGCACCCTCCCTTTCGATGATGAGCACGTGCCTACGCTCTTCA
AGAAGATCCGAGGGGGTGTGTTTTACATCCCAGACTATCTCAACCGTTCTGTCGCCACTCTGCTGATGCA
CATGCTCCAGGTGGACCCCCTGAAGCGAGCGACTATCAAAGACATACGAGAACATGAATGGTTTAAACAG
GATTTGCCCAGCTACCTATTTCCTGAAGACCCCTCCTACGATGCGAATGTCATTGACGATGAGGCTGTGA
AGGAAGTCTGTGAGAAATTCGAGTGTACAGAGTCAGAAGTGATGAATAGTCTGTATAGTGGTGACCCTCA
AGACCAGCTTGCAGTGGCTTATCATCTTATCATTGACAATCGGAGAATAATGAACCAAGCCAGTGAGTTC
TACCTCGCCTCTAGTCCTCCATCAGGTTCTTTTATGGATGACAGCGCCATGCATATTCCTCCAGGCTTGA
AACCACATCCAGAAAGGATGCCGCCTCTCATCGCAGACAGCCCCAAAGCACGCTGTCCACTGGATGCACT
CAACACAACGAAGCCCAAGTCTCTAGCTGTGAAAAAAGCCAAGTGGCATCTTGGAATCCGAAGCCAGAGC
AAAGCGTGTGACATTATGGCTGAAGTGTACCGAGCTATGAAGCAGCTGGGTTTTGAATGGAAGGTAGTGA
ATGCATACCATCTTCGAGTAAGAAGAAAAAACCCAGTGACTGGCAACTATGTGAAAATGAGCTTACAGCT
TTACCTGGTAGACAGTCGGAGCTATCTTCTGGACTTCAAAAGCATCGATGATGAGGTGGTGGAGCAGAGG
TCTGGTTCTTCAACACCCCAGCGCTCCTGTTCTGCTGCGGGCCTCCACAGAGCACGGTCAAGTTTTGATT
CCAGCACAGCTGAGAACCACTCCCTTTCTGGCTCTCTCACTGGCTCTTTGACTGGCAGCACTTTGTCCTC
GGCATCCCCGCGCCTGGGCAGTCACACCATGGATTTTTTTGAAATGTGCGCCAGTCTTATCACTGCTTTA
GCTCGTTGATTATCCACCCCTGGTCTCTGTCTTTCTGTCACCCCCCTGTGAAATCACATACACTCTTTAA
ATTATTATCTCACTCGGTATTACGTGCTCTGCAATAGAAGTTGGTGTGAACATTCCCAGGTGACATGCAG
TGTTATTGAAATCACCGAAAACACAGAAATCCAGCATTCTGTTTACTCGTAGAACTCTGTAACTCTCCTG
TGCCTGTGACAAGTTTGCATGGGTAGTACTGGGTGAGTGTGGTGGCGCTTGCTAACTTACACCTGTGAAT
TCGCTGTAGATGGCAAGCACACCTCACTGATGAACGAACCCGCTGAGGTAGGGGTGGTTCAATGGGCCTC
CTTCCTTCATGTTTCCTGTACGTAAATCCGGTTTACCTCCTAAACTTCAGCAGCTGTTAGGCTGCCTGGG
CACTCTTGCACAAGAAGATTAAGATATAGAATAACTGTTAGGGAAATATTATTTCAATGTAGAAATCTGA
AAATCCTGTCCCCCTAAATATTAGAAACCAAAGTCTTTTAAATTATTTCTGCAAATACTGCCTAGTATTA
GCCATTAAGACTGTGTTTCTGATAACAGGAGCCTAATCTCCCAGCTTCCTGACAGTTGTGCAAGGCGCCT
AGTTAAATCACACCACCACCAAGCAAAACAAAACCATGCAAAATTAGTACACAGTTCAATGAGACAAGCA
AAATACCAACAACAGTCTCTTAAAAGAAAATTCTACTCTATGATCTTGTTTGTGTTTATCAGACTGGGGT
AGCTGGGAGGAGGGCCGGCAGTCATGGCCCTCTGGCATGTTTGCTAATTCCCTTAACATTTGTTATCGAT
GCCACCTCCTCCCTCCGTCTTCCTCAGGAGCCTCTGTCTTGCTCAATGCTTTTCCCTTTCATTAATCCTT
TAGCTAAAAACCTTGCTTTGGCCATCATGTCTTTGTATACTTCCAAAGCTTGATCTCTGTGACCTTCACT
GTTGAACCTGATTGGACAGGGAAGCCTTAAATATTTAAAAGTATATTCTCCTGGAATACGTATATGTGTT
GTTTACATATATATACATATGTATTGACACATCAGTGCTTTTAATCAAAAAAAAACCAAACATTTTTGTG
CAAGTTTTTTTTTTAATTTAAGACAAAAAAGCACTATATTGCAAGATAGATTAAGTTAATTGATTATATG
TTGTTTTGGTAAAAGTATTTTAAATTATGTGTAATTTATCTAATAATTATTTATTCATCTATTGTATTTA
TGTTGTAATTTTAAAAATCATTGATCTGATTCACCCTAGAAAATATTAAGCTCTTGAAAAAAAAAAAAAG
CAACATTGCCTTGACAGGTTTCTCTTCACCAAAAATTGTATATTTTCTATTTACCAAGAAGCAAATGAGA
 
CTGTTAGACCAGGTTTCTCATGAGATTCCAGAAGACAAGTTTGTCTGTCTTTAAATAGCTTTTTGAATCA
GGAATTTGCATCATATCACATCAGATAACTTTGTAGTTCTTTTAAAAAGATGGTCAATATTTATCACTCC
AACTTCTCAATGGTAAAATAAAAGAATCTTTGCCCACTGATTGAGAAGGAGAGTGTATAATTTAAGGTAC
CTTAAGTTTAATCTTTGAAAGATTGCCATCTTGTGGCAATAGATGGTAATTTTAGTAGTTATTTTAATAC
CAATACTTAGTCCTGCCTGGGTTATCTAATGTGTTTTATGAATGTGGCATAGTTCTGACTTCTCCAAATG
TGTCTGTATCCTTGTTTGTGGGGTGGGGGAGAGATCACCTTATGATGTTTGCCTAATCATGGCATGGAAG
GCCTTGAAGTAAGGAACTGAATAGATTCTTTACCTCTAATAACAGCATCCACAACAGTGATGAAGGCAGG
AAGGAGGCAGAATGCTTTTAGTCTAGTGAATTTAAACCAAAATTACTTTATATGAATGATTATGATGCTA
TTCAAGAGCTAAAAGTTATTTACTATTGTCTTAATTCATTCCAGTATTATGAATACCCAGGTTGACAGTT
ACCTGCTGAATTTCAGGAGAACTGAAGGTCATTTTGTTTCTTTTAACATTTTAAGACATAATTATTTAAT
GAATCTAGATGATAGAGTAAGTTGGTTTTCAGAGAGAATTCAGTGACTCATCTCGAAAAGCAGAGGCAGG
AATTCAGTCACAGCATAGGGATTTGTTGCTCTTGGAAACAGAGAAGGAAAGAATTCTTTGCAAATTTACA
GTTCCTACATTTCAGTCAATCCTATGGGTATGGTAGGTACTGAGTTTAGAGCAGCCTATCCTCTTGGATC
GTGCGGAGAATAACTATTAAGTTATAGATGTGGTATGTCTTGGAAACGGAGTCCATCTGTTTCCAAAATA
GTATTTCCTAAAACAAAGAAATCCCACAAAATAATTTTGAAATACATTGTGTTCTGCTCTGCACCTTGCA
GAGGACAGTGACTATTAACTCGAAAGACTTGCAGACTTTGAGAAAAAAACAAAACAAAAAGACCCCACAT
AACTGCAAAAACCCTACTTTGCCCAAACTAGTTTACTCTTTTAAAACGTTTGCTGAGTCTGGTTGCTTAC
CTGTAATACGACTGGGGAGTATGAGGCAGGAAGATACGTTGGGGCCTAAGAACTAGATAGATAGCAAGAC
CTTGTTTCAAATTGAAACAATAAAGGAACTGTAGCATCCCATAAGAACATAGGAGAAACGATTGTCTCTG
AGCGCCACGGTGTGGTCGTTAGTGACAGTGAGTAACACCTCTTCCCCAGAGCCTGATGCTAATTCAGATC
TGCTCCATTGACCATGCACATAGGGTCATCCTGAGTCTGGGCTTGTACCACATGCTGGTTTTCCTAATCA
TATGATGCTTTTTAGGTAGAACATCTATTCTTTTTTTCCCTTAGAGGGTGACTGAAAAGTCCTAGACAAG
GTTACTTATGGAAAACCAGAAGAGTAAGAGAGCAGTGGCTTTCTGTTGTGGCTTCATGGATAGCAAGCCA
GTTTTATTCATTTTTCAATGGCCACTGAACGTTTTCTAAGTCCTTTTATTTAGGAAACCAAAATTGAAGC
TATCTCAAAGTAAATCTACAGGGAGAGAGAAAACCAAACCATTCACAAGTTAGGGTTCTGTTTTGATCAA
GAACTCTTGTAAAAGGCCCTTGCAACATCTGGTTATCTGCAGAGAGAAGCTAGGAGCCTAAGCTAAGCAG
AAACATTAAGACCCTCTGTGTTAGGGAATTGGGTATATGTAGCACAGTAATTCTCTACTTTATGCAAGTA
AGTATTCAAGAATATTTACTTAATTCTCAATCTACTCTTTTCTTAGCTAGTTTGTACTCAGGGAACACAA
ATTTAATACAAATGAAATGAAACACAATTTGGAAAATAATCGTAAACAACCGAATCAGATTTTTATACTG
CTTCATCTGTAGTCTCTGCTAACATGCAAGCATGCTTAGTTAATGTGGGCCCCACACTGATGTTCATAAG
ACTTAACAGCCTGGAACATACCTAACACAGATACCACAGCTCTGCCTGGTCACTCCCAGGGTGAAGTCTC
ATACCTAAATGTGAGACTGCCCGTAGACTTGTCTGCTTTGTGTTTGGTTCAGTCTGGCAACTTGTCCTTC
AGTTCTCTGTGGCTTGTTAAGTAGGGCTTATGGCTTAGTAGTTCTTGGCAACTCTGTTGAAAACTCTGGA
TAGTAAGTGTGATCTAGGACTTGGCCTCAATTTAAAACCCTAAAATGAAAGGGATGGACACAGAGCTATC
GTCCCTCTGAGCTCTGAAGTCATGCCTTGTTTACTACTTCAGAGCGTCAGTGTGAGTGTTCACATTTTAT
GTATTTGTTTGCTAGTATGAGTGTTTTTTCCTTAGTCCGAGGCATTTCAAAGGTACACTAACCAAAAGTA
CTTGGACTACTGGAATGCAAAACTGAAAACATGGTGTGGATTTAGCATTCATTAGAAGATGACTGCTAGG
TAGCAGTGGTGGGGCCCATGCATTTCCAGGTCTGCTAGTGCTCCCTCGCAGTACCCAGTCCAGTAAGCAG
GCTCACTGCAGTTGATCATCTTCTGGGCTGAACAGAAAGAGTTTGTGAAACATCTGAGAGTCAAGTAATA
AAAATAAACACTGGTGTGTTGTTTGTAAGTCCTGGAATGACATATAGTAGTTATAAGTGAATGTAGTTAG
TCTAATTATAAAAGCTAGTTGATTGCACTAACTAGATGTAATTTTGTGAAATATTTATGATTATATTGAT
TTTATTATAAGTATTTATAAGTTATTGAGGGTGCTTAAAATCCAGAAAAATCTCTAGGACATATTTTTTT
TTCTGAAATTGTATTTATATGATTTGGGTGTAGGGAGTTTTATACTGGTGACTGAGCCCAGGCTTCCCGT
GTAAGAAGCAAGGGCTTAACTTCAACCTCAATCCCCTGGCCCTCTACATGATTCTGAAAGTCCAGGCGCT
CTTTGTCGAGGAAAATTAGCCCCAGCCCGATCTAAATGATCACAGTTTGTGACTGCTAGCTGAACTAGGC
TTATCTAAATTTCATTTTGGCCATTTTTAATTTTGTGCATAAATGAGATGACACTGTTTTTATACAGTGT
GTCTCTCTGAGCTACCTAATCACTATTGGCTCTGATAGAAGTTCACCAAGCCTTTTTAAACTTGGAAACG
TAACTTAAGTTAATGAGCTTTAAGTCATGCATATATGTATGTGTATATATACATATGGATTATGTAAACA
AGTTAGCAGCGGCGTCTTTATCAGCGGCGCCTTTCCTTGAATATTTATGATGCGGTTGATGCTACTGAAC
AGTAATACCGTGAGGAACAGGCCCCAGTCTAGACTCTAGACTGGATGGAGTTTTGATTAAGCTTCTTCAG
CCATAACCTGTTCAGAATCAGGTCCAATCCAGACGTTATCAGACTGCAGGCCTGTATTTTGTGAGCCAAC
ATTAATGTTTACTGCCAGCCTTGCATAGCAGTGCCGGTTGCGACTGCATGTGACTTTTAGTAACTGTAGT
TTATGTCTAAAAGCAGTTCAAGTTTTATAGAGGTAATTCATGATTATTTGAACAAGCAGGGTGGGTAATT
CAAATATATTTAAAGTGCAATTAAGGAAATTCTAGGGTATAATATTCTTTCCAGTTTTAAAAAAAATAGT
 
TAAGGATTTTTAGAATGTCCTAGAATGAAAAAAATTTTTTTTGGACAGACTTTTTAAAAAACAAGGTTTA
TACAGATTTTTTTTTTTAATTCAAGGTTTGCTACTTTGTGTTTATTTAAATTTGCAAAGAGAAACCGAAG
CCTCCTAGGAAAGAGTTGCATTGTTATTTTTCTCCTGCTAGTCTGAATGTGCATTTTCACAGCTAAAAGT
CACTTGTAAATAAGGTTTGTTACCACATTCGAAGCCCATGTTTTCTAAATGGAGAACATTTTCCCAGAAT
CAAAGAGACATAGACCCACTGTCTACACAAAGAAAGAAAGCGTACCTGGTGCCTTCTCAAATAATTGTAG
AGTCAAAGTGAACGTGTTCCAAGTCTGCCCCACCCCACCCCCTCTCCTGTTCCTCGGGTTCTCTGTGACC
TACCTGTCCCTCCCACAGACGGTCACAGGATTTGCTTCTGCCCTTTCAGTTCTACTTGACCAAACCTCAC
AGCATCACCAGTGACGTTCTTCACTGAATACATTTTCTCTTTCACTTATTTCACTTTTTAAAGCCACCTG
GGAAAATATTTGGCTGCCTTCTTATGCTTTTAAAATGAATGCAATTATTAGCAGTGTTTATATTTGAAAG
TTTATAAAGGAAAGAAATTCAGAGCTAAGGACTGACTCTTAATTGGATCTAATTCCCTGTGTGTTACGAT
AGCAGCACAAGTTATGTAACCTTGTGTAGTTTTCTCTTGTACAAAATGTGGATGATAACCTACCTCACAG
ATGTGTGTGAGAAGCCGTTTCAAAGCCCCTAAGTAGGGGTTTATAGTCCCATCCTCAGTGTCAATCCTCC
CTTTTTTACTAGACACCTTTGAGAATGGTAGGATGAGTATCTTGTTTTTAGATGTCATATGTAATAAGGG
CATAGTGCTGAGAATGTTTAAGACACAGTTTCACAAATCATAGTGTTCTTACTGTAAGACACTTAAAAAT
TTCTTGAAAACCTCAAATGTTTAAATATCGAATCTGTCTTTTGTTTCAATAAATATTGTTATTCAAAGTG
TTGTCTGAAGCTGCCTTAATGTACCAAAATACAACTGTCAAAACTGTTCACAACTATATTCATGGTTGTA
CTGTTTTGAGTGTTCATTTTGAAGTTGCTATGAACTATGACCTTGAAGGATAGTATTTTGATATTATTTT
GGTAGGAATAGTTGTGGTTTTTTTTTTCATCATGTTTAACAGTGACTTTTGTGTGTAAAAAATAAAACTG
AAACTTGGCAG
**SEQ ID NO: 9:** Hsp70 promoter

CTAGAATCCCAAAACAAACTGGTTATTGTGGTAGGTCATTTGTTTGGCAGAAAGAAAACTCGAGAAA
TTTCTCTGGCCGTTATTCGTTATTCTCTCTTTTCTTTTTGGGTCTCTCCCTCTCTGCACTAATGCTCTCTC
ACTCTGTCACACAGTAAACGGCATACTGCTCTCGTTGGTTCGAGAGAGCGCGCCTCGAATGTTCGCG
AAAAGAGCGCCGGAGTATAAATAGAGGCGCTTCGTCTACGGAGCGACAATTCAATTCAAACAAGCA
AAGTGAACACGTCGCTAAGCGAAAGCTAAGCAAATAAACAAGCGCAGCTGAACAAGCTAAACAATC
TGCAGTAAAGTGCAAGTTAAAGTGAATCAATTAAAAGTAACCAGCAACCAAGTAAATCAACTGCAAC
TACTGAAATCTGCCAAGAAGTAATTATTGAATACAAGAAGAGAACTCTG

It will be understood that particular embodiments described in the examples are shown by way of illustration and not as limitations of the invention. The principal features of this invention can be employed in various embodiments without departing from the scope of the invention. Those skilled in the art will recognize or be able to ascertain using no more than routine experimentation, numerous equivalents to the specific procedures described herein. Such equivalents are considered to be within the scope of this invention and are covered by the claims.

### EXAMPLES

***Abbrevations***: 5-aminoimidazole-4-carboxamide ribonucleotide (AICAR); acetil-CoA carboxilasa (ACC); Activating Transcription Factor 6 (ATF6α); AMP-activated protein kinase (AMPK), apparent diffusion coefficient (ADC); C/EBP homologous protein (CHOP); diffusion-weighted magnetic resonance imaging (DWI), echo time (ET); endoplasmic reticulum (ER); eukaryotic translation-initiation factor 2 (peIF2α); Fatty acid synthase (FAS); field of view (FOV); flip angle (FA); Magnetic resonance angiography (MRA); Magnetic resonance imaging (MRI); Mechanistic target of rapamycin (mTOR); midline deviation (MD); multi-echo multi-slice sequence (MSME); number of averages (NA); oxygen-glucose deprivation (OGD); p70S6 kinase (pS6K), phosphorylated Unc-51 Like Autophagy Activating Kinase 1 (pULK-1); protein kinase RNA-like ER kinase (PERK); Regulatory-associated protein of mTOR (RAPTOR); repetition time (RT); spectral bandwidth (SW); T2-weighted (T2W); transient occlusion of the middle cerebral artery (tMCAO); time-of-flight MR angiography (TOF-MRA).

### 1. MATERIALS AND METHODS

### 1.1 Animals

Experimental protocols were approved by the local Animal Care Committee (Project ID 15010/14/006 and 15012/2020/010), according to the European Union (EU) rules (86/609/CEE, 2003/65/CE, and 2010/63/EU). The study conformed to Animal Research: Reporting of In Vivo Experiments (ARRIVE) guidelines. Male adult Sprague-Dawley (SD) rats and C57/BL6 mice, provided by the Centro de Biomedicina Experimental (CEBEGA); Santiago de Compostela, with a weight of 200-250 g for rats and 25 g for mice were used. Rats were housed in a temperature-controlled (22-24°C) room with a 12-hour light, 12-hour dark cycle (8:00 AM-8:00 PM). Rats were fed with water and food ad libitum. Anesthesia was induced by the inhalation of 5% sevoflurane in a N₂O/O₂ mixture (70/30). Rectal temperature was monitored and maintained at 37 °C ± 0.5 °C by using a feedback-controlled heating system. At the end of the procedures, animals were sacrificed by cervical dislocation and subsequent decapitation according to the European Union (EU) rules (86/609/CEE, 2003/65/CE, and 2010/63/EU). Brain was extracted, frozen in liquid nitrogen, and stored at 80°C until further processing.

### 1.2 Middle Cerebral Artery Occlusion

Transient focal ischemia was induced in rats by using the transient middle cerebral artery occlusion (tMCAO) model following the surgical procedures previously described [1-3]. In brief, under an operating microscope, tMCAO was performed by exposing the left common, external and internal carotid arteries through a midline neck incision. The left external carotid artery and pterygopalatine artery of the internal carotid artery were separated and ligated by 6-0 silk sutures. A silicon rubber-coated filament (403512PK5Re; Doccol Corporation; Sharon, Massachusetts, USA) was inserted through the external carotid into the left common carotid artery and advanced into the internal carotid artery to 20 mm from the bifurcation to occlude the origin of the MCA. A laser-Doppler flow probe (tip diameter 1 mm) attached to a flowmeter (PeriFlux 5000; Perimed AB; Stockholm, Sweden) was located over the thinned skull in the MCA territory (4 mm lateral to the bregma) to obtain a continuous measure of relative cerebral flow during the arterial occlusion. Once the artery occlusion had been achieved, as indicated by Doppler signal reduction, each animal was carefully moved from the surgical bench to the Magnetic Resonance Imaging (MRI) system for ischemic lesion assessment using apparent diffusion coefficient (ADC) maps (defined as T0 ischemic basal lesion). MR angiography (MRA) was also performed to ensure that the artery remained occluded throughout the MR procedure. After MR analysis, the animals were returned to the surgical bench and the Doppler probe was repositioned. The filament was removed after 75 minutes of occlusion. After suture removal, the left pterygopalatine artery was reperfused while left external carotid (used to introduce the suture) remained tied to avoid bleeding. As we have described in elsewhere [2], this surgical ischemic protocol, based on the combination of laser Doppler monitoring in combination with basal ADC and MRA assessment represents a reliable inclusion protocol during ischemic surgery to reduce variability intergroup and guarantees the reproducible of the infarct volumes. According to our reported protocol [2], the following exclusion criteria were used: i) less than 70% reduction in relative cerebral blood flow; ii) vascular abnormalities, as determined by MRA; iii) baseline lesion volume of less than 25% or greater than 45% with respect to the ipsilateral hemisphere, as measured using ADC maps, and iv) absence of reperfusion or prolonged reperfusion (more than 10 minutes until achievement of at least 50% of the baseline cerebral blood flow) after filament removal. Sham control groups were animals subjected to the same surgery procedure, but without occlusion of the middle cerebral artery (MCA).

Experimental procedures were performed following criteria derived from the Stroke Therapy Academic Industry Roundtable group guidelines for preclinical evaluation of stroke therapeutics [4,5] which are: **i)** cerebral blood flow was measured to confirm the vascular occlusion as an index of the reliability of the ischemic model; **ii)** animals were randomly assigned to the experimental groups using the GraphPad software randomization tool (www.graphpad.com); **iii)** researchers were blinded to treatment administration; **iv)** researchers were blinded to treatments during outcome assessment; and **v)** temperature was controlled during the ischemic period.

### 1.3 Experimental protocol

The study was divided into 3 steps: **a)** a comparative study between ischemic rats fed with standard diet (STD) vs. SHAM; **b)** neuroprotective study of nicotine treatment in both ischemic STD group; and **c)** we conducted a study with AICAR (5-Aminoimidazole-4-carboxamide ribonucleotide; TRC Inc., Canada) treatment, a direct activator of AMPK, in only STD group in order to elucidate that AMPK mediates protective effects of nicotine. Nicotine (nicotine-hydrogen-tartrate salt; Sigma-Aldrich; St. Louis, MO, USA) or vehicle (saline) were given subcutaneous (2 mg/kg per 12 h) in a total volume of 100 µL after one hour of reperfusion until the end of the follow-up period. During the whole period of treatment (vehicle, nicotine, or withdrawal), rats were maintained either on STD. AICAR was given subcutaneous (500 mg/Kg per 24h) after one hour of reperfusion until the end of the follow-up period (7 days). The sample size of all groups (n) was always equal to or greater than 8 animals per group.

### 1.4 Magnetic resonance imaging and image analysis

MRI studies were conducted on a 9.4 T horizontal bore magnet MRI system, Biospec 94/20USR (Bruker BioSpin; Ettlingen, Germany), with a 20 cm wide actively shielded gradient coils (440 mT/m). Radiofrequency transmission was achieved with a birdcage volume resonator; signal was detected using a four-element surface coil, positioned over the head of the animal, which was fixed with a tooth bar, earplugs, and adhesive tape. Transmission and reception coils were actively decoupled from each other. Gradient-echo pilot scans were performed at the beginning of each imaging session for accurate positioning of the animal inside the magnet bore. ADC maps were acquired during tMCAO from DWI using a spin echo echo-planar imaging sequence with the following acquisition parameters: echo time (ET) = 24.89 ms, repetition time (RT) = 4.5 s, spectral bandwidth (SW) = 200 kHz, seven b values of 0, 300, 600, 900, 1200, 1600, and 2000 s mm⁻², flip angle (FA) = 90°, number of averages (NA) = 3, 14 consecutive slices of 1 mm, 24 × 16 mm² field of view (FOV) (with saturation bands to suppress signal outside this FOV), a matrix size of 96 × 64 (isotropic in-plane resolution of 250 µm pixel⁻¹) and implemented with fat suppression option. MCA status was evaluated in a non-invasive manner with the time-of-flight MR angiography (TOF-MRA). The TOF-MRA scan was performed with a 3D-Flash sequence with an ET = 2.8 ms, RT = 15 ms, FA = 30°, NA = 2, SW= 98 kHz, 1 slice of 14 mm, 30.72 × 30.72 × 14 mm³ FOV (with saturation bands to suppress signal outside this FOV), a matrix size of 256 × 256 × 58 (resolution of 120 µm pixel⁻¹ × 120 µm pixel⁻¹ × 241 µm pixel⁻¹) and implemented without fat suppression option. The progression of ischemic lesions and infarct volumes were determined from T2 - maps calculated from T2-weighted (T2W) images acquired 24 and 72 hours, and 7, 14 and 28 days after the onset of ischemia using a multi-echo multi-slice sequence (MSME) sequence with an ET = 9 ms, RT = 3 s, 16 echoes with 9 ms echo spacing, FA = 180°, NA = 2, SW = 75 kHz, 14 slices of 1 mm, 19.2 × 19.2 mm² FOV (with saturation bands to suppress signal outside this FOV), a matrix size of 192 × 192 (isotropic in-plane resolution of 100 µm pixel⁻¹) and implemented without fat suppression option. All images were processed, and maps were constructed with imaged (https://imagei.nih.gov/ij/). Infarct volumes were determined from ADC maps and T2 relaxation maps by manually selecting areas of reduced ADC values or hyperintense T2 signal by a researcher blinded to the animal protocols [6,7]. Infarct size was indicated as the percentage of ischemic damage with respect to the ipsilateral hemispheric volume, corrected for brain edema. For each brain slice, the total areas of both hemispheres and the areas of infarction were calculated. An edema index was measured by quantifying the midline deviation (MD), calculated as the ratio between the volume of the ipsilateral hemisphere and the volume of the contralateral hemisphere. The infarct size was adjusted for edema by dividing the area of infarction by the edema index [mm³/MD]. Finally, the presented infarct volume was calculated as following: (infarct volume [mm³/MD]/ipsilateral hemispheric area [mm³]) × 100.

### 1.5 Behavioral tests

Sensorimotor deficits were assessed using the Bederson test, which is a validated scale to evaluate neurological deficits after cerebral ischemia. This test is based on the presence or absence of a reflex or spontaneous activity of the animal [8]. Bederson test was performed by a blind researcher to the treatment 1 day before MCAO, and at 24 and 72 h, as well as at days 7, 14 and 28 after ischemia induction during the darkness cycle.

The Open Field test was carried out to determine different locomotor parameters of the mice, such as speed, distance traveled and immobile times, among others. The test was recorded and analyzed using Anymaze software.

### 1.6 Western blot analysis

Protein expression was detected in rat brains by western blot. All rats were sacrificed at day 28 after MCAO, except for the rats included in the AICAR treatment protocol that were sacrificed 7 days after tMCAO for being the temporal point with the highest peak of brain lesion volume, and their brains were then processed. All mice were sacrificed at day 7 after MCAO, and their brains were then processed.

First, the portion of the brain of interest for the WB analysis was separated, and the rest of the sample was refrozen at -80 °C. Specifically, using a brain matrix, a 2 mm thick axial section was extracted from the ipsilateral hemisphere corresponding to the middle area of the ischemic lesion. After that, this ipsilateral hemisphere brain section was initially pulverized in liquid nitrogen using a mortar and pestle followed by homogenization in lysis buffer (InvitrogenTM, Life Technologies, USA) supplemented with the protease's inhibitor cocktail Complete Mini (Roche Applied Science; Switzerland). Following homogenization, the samples were then centrifuged at 12,000 rpm at 4°C for 30 min and supernatant protein concentration was quantified using the Bradford assay (Bio-Rad Protein Assay Kit, Bio-Rad, USA). The protein concentration was determined by the Bradford Method (Protein assay dye concentrate, Bio-Rad Laboratories; Hercules, CA, USA), and the total protein content of the tissues was calculated. The protein lysates were subjected to SDS-PAGE, electrotransferred to polyvinylidene difluoride membranes (PVDF; Millipore; Billerica, MA, USA) with a semidry blotter and probed with primary antibodies against acetyl-CoA carboxylase (ACC) and AMP-activated protein kinase alpha 2 (AMPKα2) (Millipore; Billerica, MA, USA); pACC, pAMPK, phospho-mammalian target of rapamycin (pmTOR), phospho-ribosomal protein S6 kinase (pS6K), phospho-regulatory associated protein of mTOR (pRAPTOR), phopho-Unc-51 like autophagy activating kinase 1 (pUlk1), Caspase-3 and cleaved Caspase-3 (Cell Signaling; Danvers; MA, USA); activating transcription factor 6 (ATF6α), phospho-PKR-like endoplasmic reticulum kinase (pPERK), phospho-eukaryotic translation initiation factor 2 alpha (peIF2α), and C/EBP homologous protein (CHOP) (Santa Cruz; Santa Cruz, CA,USA); and phospho-iron-responsive element-binding protein (pIRE) (Abcam; Cambridge, UK), or β-actin (Sigma-Aldrich; St. Louis, MO, USA) as described [9-12]. Each membrane was then incubated with the corresponding secondary antibody: anti-mouse, anti-rabbit or anti-goat (all of them from DAKO; Glostrup, Denmark), as shown [14-17]. The membranes were exposed to an X-ray film (Fujifilm; Tokyo, Japan) and developed using developer (Developer G150; AGFA HealthCare: Mortsel, Belgium) and Fixator (Manual Fixing G354; AGFA HealthCare: Mortsel, Belgium). Autoradiographic films were scanned and the bands signal was quantified by densitometry using ImageJ-1.33 software (NIH; Bethesda, MD, USA), as shown [9-12]. Values were expressed in relation to β-actin (brain). Representative images for all proteins are shown; in the case of the loading controls a representative gel is displayed, although each protein was corrected by its own internal control (β-actin), as explained above. In all the Figures showing images of gels, all the bands for each picture come always from the same gel, although they may be spliced for clarity.

### 1.7 Statistical analysis

All data were presented as the mean ± standard error of the mean (mean±SEM). One-way or two-way analysis of variance (ANOVA) followed by post-hoc Bonferroni evaluation was used for multiple groups to determine significant differences. Student's t-test was used to test the differences between two groups. Statistical significance was set at *p<0.05, **p<0.01, and ***p<0.001. The statistical analysis was conducted using GraphPad Prism v.8 for Macintosh/Windows (GraphPad, La Jolla, CA, USA). A minimal n=8 animals per group were required to detect the desired effect with a power (1-b) of 0.8 and an a of 0.05. N was calculated using EPIDAT software (http://www.sergas.es).

### 2. RESULTS

### 2.1 Nicotine treatment reduces brain ischemic injury and improves neurological recovery after cerebral ischemia

Next, we examined the potential neuroprotective effect of nicotine treatment in control (STD) ischemic rats. Notably, when ischemic brain injury was examined, our data showed that nicotine treatment induced a significant reduction of lesion volume from 24h after tMCAO until the end of the follow-up period of 28 days in the STD rats **(****Fig. 2A**, B). These neuroprotective effects on the lesion volume were also reflected in a reduction in the edema volume at 3 days after tMCAO **(****Fig. 2A**, C), as well as in a significant reduction of neurological deficit evaluated by the Bederson at day 7 after tMCAO **(****Fig. 2D****).** All these results together show that nicotine treatment exerts a neuroprotection.

### 2.2 Nicotine treatment inhibits the activation of AMPK signaling after cerebral ischemia

Similar to previous studies in the hypothalamus [11,12], STD ischemic rats treated with nicotine showed a reduction in the levels of pAMPK and its surrogate marker pACC, despite of increased expression of AMPKα2, likely due to compensation, in the extracts of brain injured area **(**Fig. 3A-H). Changes in pAMPK were associated to decreased ER stress markers levels (such as peiF2α and pIRE) in STD ischemic rats treated with nicotine. Finally, in keeping with the neurological improvement and reduction of lesion volume, nicotine treatment induced a reduction in the caspase-3 levels in both control and obese ischemic rats treated with nicotine **(**Fig. 3G-H). Altogether, these results indicated that nicotine treatment, besides beneficial therapeutic effects, also inhibits AMPK signaling, ER stress and apoptosis in the brain-injured areas induced by the ischemia.

### 2.3 The neuroprotective effects of nicotine treatment after cerebral ischemia depend on the inhibition of AMPK

The obtained evidence indicates that nicotine-induced neuroprotection after cerebral ischemia was associated with an inhibitory effect on AMPK, as well as downstream targets (ER stress), and apoptosis. Therefore, to address whether nicotine protective actions were mechanistically linked to AMPK inhibition, we treated STD ischemic rats with 5-aminoimidazole-4-carboxamide ribonucleotide (AICAR) treatment, a direct activator of AMPK [13]. Our data showed that the reduction of lesion volume induced by nicotine treatment was reversed by AICAR **(**Fig. 4A-C). Likewise, neurological deficits were also worsened after AICAR administration in nicotine-treated ischemic rats **(****Fig. 4D****).** On the other hand, western blot analyses of protein extracts on the ipsilateral hemisphere corresponded to the middle area of the ischemic lesion show that, as expected, AICAR reversed the inhibitory effects of nicotine on pAMPK **(**Fig. 5A-B) and downstream targets, such as pACC **(**Fig. 5C-D). Importantly, nicotine-induced reduction of some ER stress markers (Fig. 5E-F) and caspase 3 **(**Fig. 5G-H) were also blunted by AICAR. Interestingly, AICAR activator also modulates nicotine action on pmTOR pathway **(**Fig. 6A-B) Overall, these results indicate the activation of AMPK reverses the beneficious action of nicotine on cerebral ischemia, and consequently that AMPK is mediating the neuroprotective effect of nicotine on ischemic brain injured areas.

### 2.4 CMV-AMPKa2-DN extracellular vesicles (EVs) downregulate downstream AMPK targets in the brain

Two doses of sEVs were administered through the jugular at 4 hours and 48 hours after reperfusion of the animals. After 7 days of follow-up, the animals were sacrificed and the brains were extracted for analysis by Western Blot. A significant reduction in the infarct volumes of the mice was observed, a trend that was maintained from the first 24 h of occlusion until 7 days of follow-up **(**Fig. 7 A-C).

### 2.5 CMV-AMPKa2-DN EVs treatment improves cognitive impairment promoted by cerebral ischemia

Interestingly, as in the study of the ischemia area, a significant cognitive improvement is observed 24 hours after the first treatment. Specifically, a reduction in cognitive impairment, analyzed by the Bederson test, is observed in animals treated with CMV-AMPKa2-DN EVs compared to animals treated with empty EVs **(****Fig. 8 A)****.** Furthermore, when analyzing different locomotor parameters by the Open Field test, a deterioration was observed in all groups subjected to tMCAO compared to the Sham group. However, when analyzing the tMCAO groups, a significantly lower deterioration was observed in the CMV-AMPKa2-DN EVs animals compared to the empty EVs, both in distance traveled **(****Fig. 8 B)****,** average speed **(****Fig. 8 C)****,** maximum speed **(****Fig. 8 D)****,** such as rotations about the same body axis **(****Fig. 8 E)**

### 2.6 CMV-AMPKa2-DN EVs treatment improves cognitive impairment promoted by cerebral ischemia

When analyzing the brain extracts after 7 days, a reduction in ACC phosphorylation, a target of the AMPK pathway, was observed, indicating that systemic treatment with CMV-AMPKa2-DN EVs inhibits the activation of the AMPK pathway in the brain **(****Fig. 9 A)****.** Furthermore, a reduction of active Caspase-3 is observed in the CMV-AMPKa2-DN EVs group, reaching levels similar to those of the Sham group, unlike the group treated with Empty EVs where the levels are significantly higher **(****Fig. 9B****).**

Our data indicate that systemic treatment with CMV-AMPKa2-DN EVs inhibits the activation of AMPK in areas of ischemic brain tissue, reducing the affected area, improving the underlying cognitive impairment after ischemic, and reducing apoptotic processes derived from the injury.

### 3. REFERENCES

[1] Campos F, Sobrino T, Ramos-Cabrer P, Argibay B, Agulla J, Pérez-Mato M, et al. Neuroprotection by glutamate oxaloacetate transaminase in ischemic stroke: An experimental study. Journal of Cerebral Blood Flow and Metabolism 2011;31:1378-86. https://doi.org/10.1038/JCBFM.2011.3.
[2] Fernández-Susavila H, Iglesias-Rey R, Dopico-López A, Pérez-Mato M, Sobrino T, Castillo J, et al. Inclusion criteria update for the rat intraluminal ischaemic model for preclinical studies 2017;10:1433-8. https://doi.org/10.1242/DMM.029868.
[3] Pérez-Mato M, Ramos-Cabrer P, Sobrino, Blanco M, Ruban A, Mirelman D, et al. Human recombinant glutamate oxaloacetate transaminase 1 (GOT1) supplemented with oxaloacetate induces a protective effect after cerebral ischemia. Cell Death Dis 2014;5. https://doi.org/10.1038/CDDIS.2013.507.
[4] Recommendations for standards regarding preclinical neuroprotective and restorative drug development. Stroke 1999;30:2752-8. https://doi.org/10.1161/01.STR.30.12.2752.
[5] Philip M, Benatar M, Fisher M, Savitz S. Methodological quality of animal studies of neuroprotective agents currently in phase II/III acute ischemic stroke trials. Stroke 2009;40:577-81. https://doi.org/10.1161/STROKEAHA.108.524330.
[6] Reith W, Hasegawa Y, Latour LL, Dardzinski BJ, Sotak CH, Fisher M. Multislice diffusion mapping for 3-d evolution of cerebral ischemia in a rat stroke model. Neurology 1995;45:172-7. https://doi.org/10.1212/WNL.45.1.172.
[7] Verheul H, Berkelbach van der Sprenkel J, Tulleken C, Tamminga K, Nicolay K. Temporal evolution of focal cerebral ischemia in the rat assessed by T2-weighted and diffusion-weighted magnetic resonance imaging. Brain Topogr 1992;5:171-6. https://doi.org/10.1007/BF01129046.
[8] Bederson J, Pitts L, Tsuji M, Nishimura M, Davis R, Bartkowski H. Rat middle cerebral artery occlusion: evaluation of the model and development of a neurologic examination. Stroke 1986;17:472-6. https://doi.org/10.1161/01.STR.17.3.472.
[9] Milbank E, Dragano NR v., González-García I, Garcia MR, Rivas-Limeres V, Perdomo L, et al. Small extracellular vesicle-mediated targeting of hypothalamic AMPKα1 corrects obesity through BAT activation. Nature Metabolism 2021 3:10 2021;3:1415-31. https://doi.org/10.1038/s42255-021-00467-8.
[10] Seoane-Collazo P, Romero-Picó A, Rial-Pensado E, Liñares-Pose L, Estévez-Salguero A, Ferno J, et al. κ-Opioid Signaling in the Lateral Hypothalamic Area Modulates Nicotine-Induced Negative Energy Balance. Int J Mol Sci 2021;22:1-13. https://doi.org/10.3390/IJMS22041515.
[11] Seoane-Collazo P, Liñares-Pose L, Rial-Pensado E, Romero-Picó A, Moreno-Navarrete JM, Martínez-Sánchez N, et al. Central nicotine induces browning through hypothalamic κ opioid receptor. Nat Commun 2019;10. https://doi.org/10.1038/s41467-019-12004-z.
[12] Seoane-Collazo P, Martinez De Morentin PB, Fernø J, Diéguez C, Nogueiras R, López M. Nicotine improves obesity and hepatic steatosis and ER stress in diet-induced obese male rats. Endocrinology 2014;155:1679-89. https://doi.org/10.1210/EN.2013-1839.
[13] Martinez De Morentin PB, Whittle AJ, Fernra J, Nogueiras R, Diéguez C, Vidal-Puig A, et al. Nicotine induces negative energy balance through hypothalamic AMP-activated protein kinase. Diabetes 2012;61:807-17. https://doi.org/10.2337/db11-1079.
[14] Yu H, Wang X, Kang F, Chen Z, Meng Y, Dai M. Propofol attenuates inflammatory damage on neurons following cerebral infarction by inhibiting excessive activation of microglia. Int J Mol Med 2019;43:452-60. https://doi.org/10.3892/IJMM.2018.3974.
[15] Yu SJ, Wu KJ, Bae E, Wang Y -Syuan, Chiang CW, Kuo LW, et al. Post-treatment with Posiphen Reduces Endoplasmic Reticulum Stress and Neurodegeneration in Stroke Brain. IScience 2020;23:100866. https://doi.org/10.1016/J.ISCI.2020.100866.
[16] Andersen KK, Olsen TS. The Obesity Paradox in Stroke: Lower Mortality and Lower Risk of Readmission for Recurrent Stroke in Obese Stroke Patients: Https://DoiOrg/101111/ljs12016 2013;10:99-104. https://doi.org/10.1111/IJS.12016.
[17] Chaudhary D, Khan A, Gupta M, Hu Y, Li J, Abedi V, et al. Obesity and mortality after the first ischemic stroke: Is obesity paradox real? PLoS One 2021;16:e0246877. https://doi.org/10.1371/JOURNAL.PONE.0246877.

## Claims

1. A population of small extracellular vesicles (sEVs) comprising at least one polynucleotide comprising a gene encoding a dominant negative AMP-activated protein kinase alpha 2 (AMPKα2-DN) mutant protein.

2. The population of small extracellular vesicles according to claim 1, wherein the amino acid sequence of the AMPKα2-DN mutant protein comprises SEQ ID NO: 1, or a sequence with at least 85% sequence identity to SEQ ID NO: 1, with the proviso that the amino acid residue in position number 157 in SEQ ID NO: 1 is not an aspartic acid.

3. The population of small extracellular vesicles according to claim 2, wherein the amino acid residue in position number 157 in SEQ ID NO: 1 is an alanine.

4. The population of small extracellular vesicles according to claim 1, wherein the amino acid sequence of the AMPKα2-DN mutant protein comprises SEQ ID NO: 2, or a sequence with at least 85% sequence identity to SEQ ID NO: 2, with the proviso that the amino acid residue in position number 169 in SEQ ID NO: 2 is not an aspartic acid.

5. The population of small extracellular vesicles according to claim 4, wherein the amino acid residue in position number 169 in SEQ ID NO: 2 is an alanine.

6. The population of small extracellular vesicles according to any one of claims 1 to 5, wherein the gene encoding the AMPKα2-DN mutant protein is operably linked and under the control of a ubiquitous and constitutive promoter, preferably a cytomegalovirus promoter.

7. The population of small extracellular vesicles according to any one of claims 1 to 6, the sEVs express in their outer membrane at least one fusion protein comprising the neurotrophic rabies virus (RVG) peptide fused to lysosome-associated membrane protein 2b.

8. The population of small extracellular vesicles according to claim 7, wherein the fusion protein comprising the neurotrophic rabies virus (RVG) peptide fused to lysosome-associated membrane protein 2b comprises SEQ ID NO: 5, or a sequence with at least 85% sequence identity to SEQ ID NO: 5.

9. The population according to any of claims 1 to 8, wherein the small extracellular vesicles have a size distribution of between 30 and 150 nm.

10. The population according to any of claims 1 to 9, wherein the small extracellular vesicles are exosomes.

11. A population of small extracellular vesicles as defined in any of claims 1 to 10, for use in therapy.

12. A population of small extracellular vesicles as defined in any of claims 1 to 10, for use in the treatment or prevention of ischemia or ischemia-reperfusion.

13. The population of small extracellular vesicles for use according to claim 12, wherein the ischemia is a ischemic stroke or cerebral ischemia.

14. The population of small extracellular vesicles for use according to any one of claims 11 to 13, wherein the administration of said small extracellular vesicles is via systemic route.

15. The population of small extracellular vesicles for use according to any one of claims 11 to 14, wherein the use of the small extracellular vesicles, preferably exosomes, is in combination with nicotine, wherein the combination is herein understood as the simultaneous or sequential administration, in any order, of a) the population of small extracellular vesicles, and b) nicotine.
